(19) Europäisches Patentamt
European Patent Office
Office européen des brevets

(11) **EP 3 367 883 B1**

(12) **EUROPEAN PATENT SPECIFICATION**

(45) Date of publication and mention
of the grant of the patent:
**13.03.2019 Bulletin 2019/11**

(21) Application number: **16787764.6**

(22) Date of filing: **18.10.2016**

(51) Int Cl.:
*A61B 5/00* (2006.01)          *A61B 5/11* (2006.01)
*G08B 21/04* (2006.01)

(86) International application number:
**PCT/EP2016/074948**

(87) International publication number:
**WO 2017/071988 (04.05.2017 Gazette 2017/18)**

(54) **MONITORING ACTIVITIES OF DAILY LIVING OF A PERSON**

ÜBERWACHUNG VON AKTIVITÄTEN DES TÄGLICHEN LEBENS EINER PERSON

SURVEILLANCE DES ACTIVITÉS QUOTIDIENNES D'UNE PERSONNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2015 EP 15191841**

(43) Date of publication of application:
**05.09.2018 Bulletin 2018/36**

(73) Proprietor: **Koninklijke Philips N.V.**
**5656 AE Eindhoven (NL)**

(72) Inventors:
• **TEN KATE, Warner Rudolph Theophile**
**5656 AE Eindhoven (NL)**

• **FENG, Jinghan**
**5656 AE Eindhoven (NL)**

(74) Representative: **de Haan, Poul Erik**
**Philips International B.V.**
**Philips Intellectual Property & Standards**
**High Tech Campus 5**
**5656 AE Eindhoven (NL)**

(56) References cited:
**US-A1- 2005 131 736     US-A1- 2006 055 543**
**US-A1- 2009 066 501     US-A1- 2012 053 472**

**Description**

FIELD OF THE INVENTION

This invention relates to monitoring activities of daily living (ADLs) of a person and more particularly to detecting behavior which can be used to trigger a warning or intervention.

BACKGROUND OF THE INVENTION

Functional assessment or monitoring of a person's health status, physical abilities, mental abilities, or recuperation after injury, hospitalization and treatment is of primary concern in most branches of medicine, including geriatrics, rehabilitation and physical therapy, neurology and orthopedics, nursing and elder care.

Investigations have found that an individual's functional ability is actually environment-specific, since function increases when subjects are in familiar surroundings due to reduced confusion. Also, one-time assessment of function does not allow for assessment of variability of functional performance over the course of a day or several days, nor does it allow for assessment of change which is important in determining the adequacy of certain clinical services and treatments (such as rehabilitation) following functional loss.

A consensus therefore exists that it is preferable to assess or monitor independent functioning of a person at their home or within familiar surroundings.

A level of independent function is commonly indicated by the quality with which Activities of Daily Living (ADLs) are performed. ADLs refer to the most common activities that people perform during a day. Therefore, a reduced quality in the ADLs can be an indicator for care needed. For example, an anomaly in the regular performance of one or more ADLs can serve as a warning for special attention.

ADL monitoring is for example of interest for patients with a range of neurological deterioration problems, like dementia. Problems are always associated with cognitive decline, progressive disorganization, temporospatial disorientation trouble and therefore difficulties to perform activities of daily living without assistance. Disturbances of some circadian rhythms like sleep/wakefulness, rest/activity cycles are also components of the behavioral symptomatology and are the main determinant of care givers' burden and entrance in an institution.

Policies which enable elderly people to stay at home or age in place are of increasing interest and the subject of research in a growing number of countries. In return, the prevalence of age-related diseases has grown in elderly people ageing in place, creating an increased need in infrastructures and medical caregivers at home.

Devices and systems have been developed to monitor the ADLs of individuals as they live independently in their own home or within familiar surroundings. For example, one such known system for detecting activities of daily living of a person system is described in US 2009/0066501 A1 and comprises three main components: (i) a sensor system that collects information about the person's activities and behaviors; (ii) an intelligence (or information processing) system that interprets the sensor signals for determination of ADL behavior; and (iii) a user interface system that enables care givers to inspect the interpreted (processed) information. The intelligence system typically makes use of computational techniques known in the art as artificial intelligence. The system may be supported by conventional technologies for data collection, transmission, and storage.

In practice, however, a major difficulty is encountered by the wide range of variations that can happen in actual care cases. For example, people can live in differently architected houses, have different lifestyles and habits. Care givers may also have different needs, locations and/or lifestyles. Also, different people may have different care needs and so differing aspects of the activities and behaviors may be of interest for monitoring. Since there are so many possible circumstances, situations and contexts that can occur in daily life, it is difficult to capture any display information about them all in a manner which is quick and easy to interpret. Quick and easy interpretation is of paramount importance in providing good quality care by professional care institutions as well as personal care givers.

A known way to provide an alert in respect of a subject is to analyze ADL data to look for anomalies. There are several existing methods to detect whether a current ADL pattern deviates from the usual one. However, a current pattern may deviate because the user has deviated from his usual routine while still performing all the activities. The order of execution, and possibly the duration of each of them, may be altered, but without the need for an alert. While methods exist to allow for extending and shrinking the time used for an activity, no solution is available to allow for permutations in the pattern.

In realistic situations a user may change the order of activities, for example swapping the order between shower and breakfast. Such a swapping should not be counted as a deviation.

There remains a need to be able to detect anomalies in a person's behavior in a way which is simple to understand and simple to implement.

SUMMARY OF THE INVENTION

The invention aims to at least partly fulfill the aforementioned needs. To this end, the invention provides devices, systems and methods as defined in the independent claims. The dependent claims provide advantageous embodiments.

Examples in accordance with one aspect of the invention provide an activity of daily living, ADL, monitoring system for monitoring ADLs of a person within an environment, wherein the ADL monitoring system comprises:

a set of sensors each adapted to respond to an activity and to generate a sensor output signal representative of the activity;

a data processing unit adapted to receive the sensor output signals and to process the sensor output signals, to:

generate an activity density map which identifies the level or type of a particular activity within particular timeslots;
generate a reference map which indicates a reference value or range of values of activity levels or types within the particular timeslots;
compare the level or type of a particular activity in the individual timeslots of the activity density map with the reference spread of activity levels or types in the corresponding timeslots of the reference map;
determine a size of correspondence of the level or type of activity arising in each timeslot of the activity density map reference spread of activity levels or types in the corresponding timeslots of the reference map to identify initial anomaly points;
for the initial anomaly points, perform a test of activity permutations to find timeslots of the activity density map which may be reordered to remove as many of the initial anomaly points as possible; and
identify the remaining anomaly points as a first anomaly indication.

The time period over which the activity density map and reference map are compared for example comprises a set of complete days. For example a complete activity density map may comprise a matrix of activity levels or types, where each row represents the time period of the reference map (e.g. a day, or certain days, or other duration such as a week, or a week from which certain days are excluded) and each column represents a timeslot within that time period.

The reference map is created based on the combination of a set of previous time periods, i.e. multiple rows of the complete activity density map. For example, the reference map may be considered to be a reference day which indicates the probability of different activity levels or types at each timeslot within that day.

The reference value or range of values may be a single, scalar number, or a binary value or a spread of values (scalar, vector or multidimensional).

The reordering of timeslots may comprise interchanging timeslots, for example simply swapping of pairs of timeslots or more complicated reallocation of timeslots..

The system as defined above is for testing of the activity density map for a day (or whatever other time period is selected as the duration of one row of the complete activity density map) against the reference day. For example, the most recent day may be selected, for testing against the reference day. The reference day may be based on days before the current day, back in time to 30 days back for example. However, some outliner days may be excluded when formulating the reference day. The number of days used to from the reference day may vary. In a first comparison, corresponding timeslots may be tested against each other, timeslot k of the test/current day being tested against timeslot 1 of the reference day, where k equals 1. The permutation allow to permute the testing: k is different from 1 (but possibly in the neighborhood), which means that the timeslot in the reference day that equals 1 is to be tested with another timeslot in the reference day (e.g. k).

This system is able to detect anomalies at the level of individual timeslots, and the permutation approach makes the system robust to changes in the order in which activities are carried out by a subject. Thus, the invention provides a solution which is able to detect anomalies in daily activity patterns, while allowing the order of activities to permute. The system is for example based on forming a matrix of possible matches between a current activity pattern (i.e. the activity density map for a particular time period, e.g. one day) and a reference pattern (i.e. the reference map) formed by the reference spread of activity levels or types. The matrix is then used to determine the correspondence defined above. Each row in the matrix for example represents a timeslot of the current time period (e.g. day, i.e. the measurement day), and each column represents a timeslot of the reference time period (e.g. the reference day). By performing the permutation process, for example moving a permutation window over the diagonal of the matrix, the number of non-matches along the diagonal is minimized. If the minimized number of non-matches passes a threshold an anomaly alert may then be generated.

The permutation test may be applied only locally around the initial anomaly points, so that it looks for local reordering of the activities rather than looking for reordering of the activities of the entire time period represented by the activity density and reference maps.

There are different ways to implement the permutation process. Essentially it looks to see if a localized (in time) change in the order in which activity types or levels are carried out can make the activity density map better match the reference map. Note that different choices of permutation may result in different remaining anomaly points. Thus, there may be multiple ways to achieve the same number of remaining anomaly points.

The data processing unit may be adapted to perform the test of activity permutations by:

setting a time window centered on an initial anomaly;
testing for reordering of timeslots within the time window which remove the initial anomaly;
determining whether or not the timeslot reordering creates new anomalies.

If new anomalies are created, a different candidate swap may then be used.

The data processing unit may be adapted to perform the test of activity permutations by recursively testing timeslot swaps within the time window to find the minimum remaining number of anomaly points for the time window.

The data processing unit may be adapted to determine the size of correspondence by determining a probability value of the activity level arising in each timeslot of the activity density map based on the reference map, and is adapted to optimize the total probability.

Thus, it is tested if actual activity levels or types are likely based on the information conveyed by the reference map and the timeslots are reordered to optimize the total probability.

The data processing unit may be adapted to generate the reference map as a sequence of activity probability distributions for each timeslot. In this way, it is possible to determine if an activity level which arises in activity density map is likely or unlikely having regard to the reference map.

The data processing unit may be adapted to form a recurrence plot from the sequence of activity probability distributions, and identify the initial anomaly points as missing points from the main diagonal of the recurrence plot. The recurrence plot provides one way to apply a probability threshold. The initial anomaly points represent timeslots for which the activity level does not seem consistent with the reference map.

The data processing unit may be adapted to identify timeslots which during the whole of the activity density map correspond to initial anomaly points, and provide a second anomaly indication based on the identified timeslots.

This may arise if there is no or excessive activity during a time slot. This can be used as another flag for an anomaly.

The data processing unit may obtain an average activity density for the activity density map, and compare the average activity density with the average activity density of the reference map, and provide a third anomaly indication based on the comparison. This may arise if there is a general reduction in activity across a full day. This can be used as another flag for an anomaly.

The data processing unit may be adapted to perform anomaly analysis based on vector analysis of the first second and third anomaly indications.

A number of between 25 and 35 activity density bins may be used in the first reference pattern and/or the number of days in the set of complete days used to form the first reference pattern may be between 15 and 25 and/or the number of timeslots used to represent a day may be between 30 and 60. These parameters provide a compromise between the reliability of the data and the processing requirements. The first reference pattern and the activity density map for example relate to individual days.

By way of example, the set of sensors may comprise one or more of:

PIR sensors;
open/close sensors;
power sensors;
pressure sensors (mats)
radar and ultra-sound based sensors
humidity sensors;
CO2 sensors;
temperature sensors;
microphones;
cameras;
wearable sensors (such as accelerometers, gyroscopes etc., heart-rate monitors, respiration sensors, body temperature sensors, skin conductivity sensors, blood pressure sensors, sugar level detectors, etc.).

Examples in accordance with another aspect of the invention provide a method of monitoring ADLs of a person within an environment, comprising:

receiving sensor output signals from a set of sensors each adapted to respond to an activity and to generate a sensor output signal representative of the detected activity;
processing the sensor output signals, to:

generate an activity density map which identifies the level or type of a particular activity within particular timeslots;
generate a reference map which indicates a reference value or range of values of activity levels or types within the particular timeslots;
compare the level or type of a particular activity in the individual timeslots of the activity density map with the reference spread of activity levels or types in the corresponding timeslots of the reference map;
determine a size of correspondence of the level or type of activity arising in each timeslot of the activity density map with the reference spread of activity levels or types in the corresponding timeslots of the reference map to identify initial anomaly points;
for the initial anomaly points, perform a test of activity permutations to find timeslots of the activity density map which may be reordered to remove as many of the initial anomaly points as possible; and
identify the remaining anomaly points as a first anomaly indication.

The method may comprise performing the test of activity permutations by:

setting a time window centered on an initial anomaly;
testing for reordering of timeslots within the time window which remove the initial anomaly;
determining whether or not the timeslot reordering creates new anomalies.

It can thus be investigated if new anomalies arise or if the number of initial anomalies has been successfully reduced.

Performing the test of activity permutations may be carried out by recursively testing timeslot swaps within the time window to find the minimum remaining number of anomaly points for the time window. A backtracking scheme instead of a recursive scheme may be used. Other schemes may be used as well, as they are known in the art.

The method may comprise determining the size of correspondence by determining a probability value of the activity level arising in each timeslot of the activity density map based on the reference map, and optimizing the total probability.

The method may comprise:

generating the reference map as a sequence of activity probability distributions for each timeslot;
forming a recurrence plot from the sequence of activity probability distributions; and
identifying the initial anomaly points as missing points from the main diagonal of the recurrence plot.

Timeslots may be identified which during the whole of the current activity density map correspond to initial anomaly points, and a second anomaly indication is provided based on the identified timeslots. An average activity density may also be obtained for the current activity density map, and the average activity density compared with the average activity density of the first reference pattern, and provide a third anomaly indication based on the comparison. Anomaly analysis can then be based on vector analysis of the first second and third anomaly indications.

The invention may be implemented by a computer program.

BRIEF DESCRIPTION OF THE DRAWINGS

Examples in accordance with aspects of the invention will now be described in detail with reference to the accompanying schematic drawings, in which:

Figure 1 shows in outline form a method of detecting anomalies in ADL data;
Figure 2 illustrates anomalies in a simple 2-dimensional data set;
Figure 3 shows two examples of a recurrence plot;
Figure 4 shows an image of an activity density map;
Figure 5 shows a reference day calculation process used for histogram based anomaly detection;
Figures 6(a) to (c) show a generation process for a normalized 30-bin ADM probability distribution histogram for one timeslot t;
Figures 7(a) and (b) shows two examples of recurrence plot;
Figure 8 shows the recurrence plot of Figure 7(b) with missing recurrence points in the upward diagonal line identified;
Figure 9 is used to explain a permutation test applied to one missing point;
Figure 10 shows a permutation test process based on simple calculations;

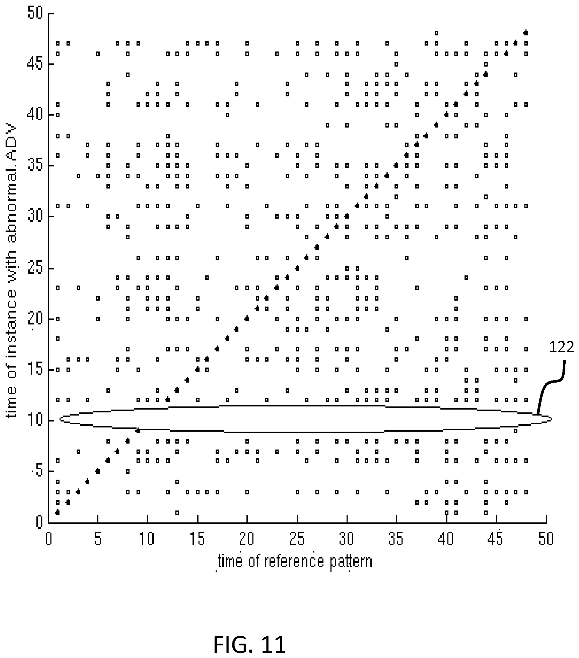

Figure 11 shows an activity density plot with no recurrence points in a particular row, showing an uncommon activity density;

Figure 12 shows the overall system; and

Figure 13 shows a computer suitable for implementing the method.

DETAILED DESCRIPTION OF THE EMBODIMENTS

An ADL monitoring system uses a set of sensors each adapted to respond to an activity and to generate a sensor output signal representative of the detected activity level or type. An activity density map is formed. The activity level or type is compared with a range of activity levels or types represented in a map which characterized a reference spread of activity levels over the same time period as the activity density map. A probability analysis is then used to identify initial anomaly points. For these the initial anomaly points, a test of activity permutations is carried out to find timeslots in the activity density map which may be reordered to remove the initial anomaly points. In this way, anomalies at the level of individual timeslots can be identified, and the permutation approach makes the system robust to changes in the order in which activities are carried out by a subject.

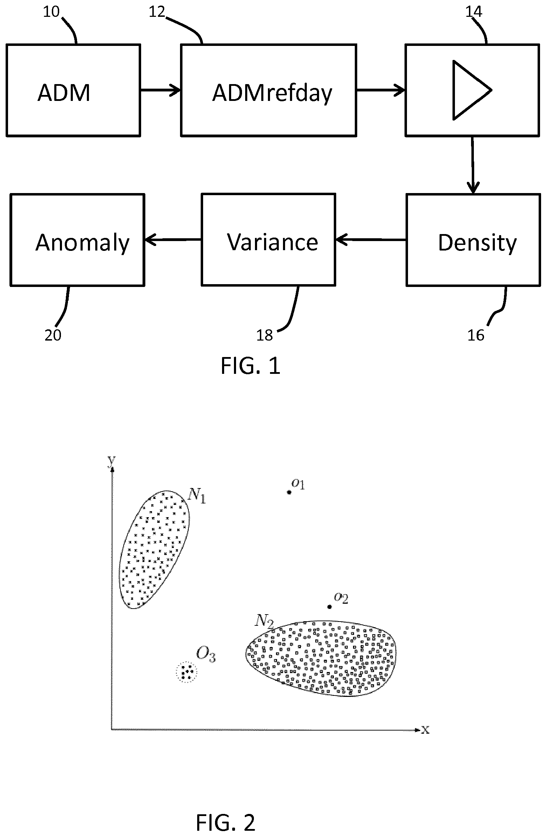

Figure 1 shows an outline of an overall algorithm process within which the approach of the invention is employed. The details are discussed further below.

In step 10 an activity density map (ADM) is generated. There can be multiple sensors in a house, and the designer of the algorithm selects the set of sensors that will be monitored for anomalous patterns. The activity density map may be based on raw sensor data or on processed sensor data. It may be prepared in any form, and thus more generally comprises a dataset of values. For example, the time sequence of subsequent rooms in which the user is present or the detected movement times are taken as input signal. The signals of the selected sensors are structured in a matrix of daily patterns, called the activity density map in which each row indicates a day, and each column indicates a timeslot of a day.

A complete activity density map has multiple rows, each corresponding to a particular time period, and each time period is divided into timeslots. However, the term "activity density map" is also used below to indicate at least one such row. Indeed, in the method and system described below, a comparison of one row (i.e. a single time period e.g. day) is made to a reference map, (which itself is also one row so that a comparison element by element is made). This one row is termed an "activity density map". The term should be understood accordingly. There may instead be comparison of multiple rows, for example a reference map of a week may have seven rows. Indeed the concept of a row is simply for ease of understanding, and the activity density map is simply a set of data entries however they are arranged.

The values in the ADM are termed "activity levels". It will be appreciated that an "activity" and an "activity level" in this context are generic terms, i.e. not necessarily reflecting physical activity but can be any form of action or event. For example, some activities have a discrete value and others have a binary value. An "activity" may even simply refer to a parameter such as a temperature level. Examples of activities are reading the newspaper, watching TV, preparing a meal, sitting, having a nap, sleeping etc. So, in particular, an activity level may be of a higher level of abstraction such as a particular ADL being performed (dressing, having breakfast, personal care, toilet visit, etc.).

In step 12 a reference day is generated from the complete ADM, or a selected part of the ADM (for example, last 30 rows, of which possibly some outliners are excluded). This can be a scalar series, with one value per timeslot, for example by averaging the values at that time slot over all days. Preferably a vector value is used per timeslot, representing the (probability) distribution of possible values. The distribution can be determined by building histograms of the values at that time slot over a set of previous days. Together, an average pattern is constructed, namely a sequence of distributions, which is called the reference day. More precisely, by normalizing each histogram, the reference day holds a sequence of activity probability distributions for each timeslot.

In step 14, the signals measured during a measurement day are compared with the reference day. For a given measurement day, the activity pattern is taken and the probability to correspond with a timeslot in the reference day is determined. This is done by table-lookup in the obtained normalized histograms. The probability listed in the histogram for the activity value computed for the current day is taken as a measure of the correspondence between the current and the reference day. A so-called recurrence plot is then derived. The timeslots of the measurement day are listed on one axis (e.g. vertically), and those on the reference day are listed on another axis (e.g. horizontally), and each probability is derived at the corresponding row-column entry. This leads to a matrix of values, called the recurrence plot.

For the sake of explanation, the probability values are quantized to binary values. The probability is quantized to 1, when the probability exceeds a threshold, and otherwise it is listed as a 0. This threshold is referred to below as the recurrence threshold. For a normal day, the diagonal of the recurrence plot should list all 1s. A 0 indicates that the activity in the corresponding timeslot deviates significantly from the reference (the probability to match is less than the threshold).

A further algorithm is applied to these points, performing a test to find out whether an activity permutation at that time with an activity at another time can compensate the found deviation. This may be a recursive process. If such permutation has been found, the timeslots are interchanged. If there is no permutation found, the 0 on the diagonal stays and the timeslot is considered to be an anomaly.

The test of activity permutations involves setting a time window centered on an initial anomaly. There is then testing for swaps of timeslots within the time window which remove the initial anomaly. This equates to considering if a locally different order of activities or events would make the detected behavior match better the reference day. The timeslot swaps may however create new anomalies, in which case the swap has not been successful. The test of activity permutations may thus be made by recursively testing timeslot swaps within the time window to find the minimum remaining number of anomaly points for the time window. A backtracking routine may equally be applied.

In step 16 an uncommon density value is identified. A second metric is defined of the uncommon density value of the measurement day. In a recurrence plot, it may happen that a fully empty horizontal line (line of 0s) appears in the recurrence plot. Such an empty horizontal line indicates a single timeslot whose density value is out of the range of the reference day. There does not exist a permutation to reduce the number of anomalies along the diagonal (for this time slot).

In step 18 a day density variance is derived. This is a third metric. It comprises the average activity density of the measurement day, and provides an assessment how off target it compares to the average activity density of the reference day.

In step 20 anomaly analysis is carried out based on the three metrics. The three metrics provide a feature vector for each timeslot. Anomaly diagnosis is based on feature vector analysis, as will be explained below.

Anomaly detection refers to the problem of finding patterns in data that do not conform to the expected behavior. It is an important problem which relates to diverse research areas and application areas, such as fraud detection for credit cards, insurance or health care, intrusion detection for cyber security, fault detection in safety critical systems, and military surveillance of enemy activities.

Anomalies are patterns in data that are considerably different than the remainder of the data corresponding to normal behavior. They are also referred to as outliers, novelties, noise, deviations and exceptions. Figure 2 illustrates anomalies in a simple 2-dimensional data set. The data have two normal regions, $N_1$ and $N_2$, since most observations lie in these two regions. Points that are sufficiently far away from the regions, e.g., points $o_1$ and $o_2$, and points in region $o_3$, are anomalies. They can be caused by instrument error, natural deviations in populations, human error, fraudulent behavior, changes in behavior of systems or faults in systems.

The nature of a detected anomaly is a significant aspect in anomaly detection. They can be categorized into: point anomalies, contextual anomalies and collective anomalies.

Point anomalies, are the most common anomalies in research, as well as simple. If an individual data is diagnosed as different from other data instances, it is referred to as point anomaly. For contextual anomalies, data instances are defined by a context attribute and behavioral attributes. The first one indicates the neighborhood (context) for that instance and the latter one captures its non-contextual characteristics. If a data instance is anomalous in a specific context, but its behavioral attributes might be normal, it is termed a contextual anomaly. Contextual anomalies have been most commonly explored in time-series data and spatial data. There can be some individual data instances in a collective anomaly that are not anomalous by themselves, however, if a collection of related data instances is anomalous with respect to the entire data set, it is termed as a collective anomaly

Anomaly detection always associates with data labeling, to denote that the data is normal or anomalous. Typically, anomalies are reported in two manners: scores and labels. Scoring techniques assign an anomaly score to each instance in the test data depending on the degree to which that instance is considered an anomaly. Thus, the output of such techniques is a ranked list of anomalies. An analyst may choose to either analyze the top few anomalies or use a cutoff threshold to select the anomalies. Labeling techniques provide binary values (normal or anomalous) to each test instance. Though it doesn't directly allow the analyst to make such a choice like scoring, it can be controlled indirectly through parameter choices within each technique.

A wide variety of anomaly detection techniques have been specifically developed for certain application domains, while some are more generic. They are generally categorized into classification based, nearest neighbor based, clustering based, and statistical techniques. Temporal sequence comparison and statistical techniques are considered most applicable in ADL data.

There exist several methods to compare two temporal sequences. The simplest way is the direct comparison on a sample-per-sample basis. A sequence distance is defined, which is usually the Euclidean distance. For instance, if $S_1$ and $S_2$ are two temporal sequences with the same length n, their distance d follows as:

$$d(S_1, S_2) = \sqrt{\sum (S_1(i) - S_2(i))^2} \ (i = 1, 2, \ldots n) \qquad \text{(Eq. 1)}$$

The distance represents the dissimilarity between the sequences $S_1$ and $S_2$. If $S_1$ represents the normal sequence (which in the context of this application is the reference day), then $d(S_1, S_2)$ indicates the anomaly score of $S_2$.

Quite often the time series will have a similar shape but differ (slightly) in local duration. In one sequence the shape might be a little more stretched at one point than in the other, while it might be compressed at another point. The Euclidean distance will penalize this stretching and compressing, causing the dissimilarity score to raise.

A method that accounts for such local stretching effects is called dynamic time warping (DTW), well known in the art. DTW calculates an optimal match between sequences $S_1$ with length $I$ and $S_2$ length $J$ and allows stretching and shrinking.

Firstly, a $I * J$ matrix is constructed, where the ($i^{th}$,$j^{th}$) corresponds to the squared distance, $d(a_i,b_j) = (a_i-b_j)^2$. A path through the matrix that minimizes the total cumulative distance is searched:

$$DTW(S_1, S_2) = min\{\sqrt{\sum_{k=1}^{K} w_k}\} \qquad \text{(Eq. 2)}$$

In this equation, $w_k$ stands for the matrix element $(i,j)_k$ that also belongs to $k^{th}$ elements of the warping path W. The warping path can be found using dynamic programming by evaluating the equation:

$$\gamma(i,j) = d(q_i, c_j) + min\{\gamma(i-1, j-1), \gamma(i-1, j), \gamma(i, j-1)\} \qquad \text{(Eq. 3)}$$

Where $d(q_i,c_j)$ is the distance found in current $I * J$ matrix, and $\gamma(i,j)$ is the sum of $d(q_i,c_j)$ and the minimum cumulative distances from the three adjacent cells.

As will be discussed below, in the system described, the reference day is computed as a sequence of probability density functions. In order to apply DTW, a distance metric for this data type is needed. One way is to replace each timeslot with the mean value, or some other central value (median, mode), of the distribution and to take the (absolute) difference between the so-constructed sequences. Another way could be to use the probability value for the activity level measured during the measurement day, where high probability has to be converted into low distance, for example by taking the complement (1-prob). However, the sequence comparison method still cannot solve the permutation problem.

In statistical techniques, an anomaly is an observation which is suspected of being partitioned or wholly irrelevant because it is not generated by the stochastic model assumed. Normal data instances occur in high probability regions of a stochastic model, while anomalies occur in the low probability regions of the stochastic model. For a given data set, a statistical model is made to represent normal behaviors. For a test data evaluation, the learnt statistical model is applied. If it is calculated to be in low probability, it is determined to be an anomaly, otherwise, it is considered to be normal.

The statistical technique is the main element in the algorithm described in this application, and this technique will now be discussed in more detail.

There are various approaches to create a statistical model. They are categorized into parametric and non-parametric. Parametric statistical modeling assumes general knowledge of the underlying distribution and estimates the parameters characterizing that distribution of the given data. It calculates the probability density function $f(x,\theta)$, where x is an observation. The anomaly score of a test instance (or observation) x is the inverse of the probability density function $f(x,\theta)$. Conversely, non-parametric techniques do not generally assume knowledge of the underlying distribution and aim to estimate that distribution from the given data.

Examples of parametric statistical modeling are: Gaussian Model, Regression Model and Mixture of Parametric Distributions Based Model.

In the Gaussian Model the data distribution is assumed to follow a Gaussian distribution, which is a very common continuous probability distribution in probability theory. For a given data set $\{x^1, x^2 ... x^m\}$, each $x \in R^n$, the mean and variance are estimated. The data are assumed to adhere the Gaussian distribution, so the estimated $\mu$, $\sigma$ (mean and standard deviation) are taken to represent the data distribution as:

$$\varphi_{\mu,\sigma^2}(x) = \frac{1}{\sigma\sqrt{2\pi}} e^{-\frac{(x-\mu)^2}{2\sigma^2}} \qquad \text{(Eq. 4)}$$

When there are several factors that need to be taken into consideration, like $x_1, x_2, ... x_h$, the total distribution can be calculated by multiplying the respective (Gaussian) distribution of each factor:

$$\varphi_{\mu,\sigma^2}(x) = \prod_{j=1}^{h} \varphi_{\mu,\sigma^2}(x_j) \qquad \text{(Eq. 5)}$$

Multiplication implies the factors are (assumed to be) mutually independent.

For a test data instance x, $\varepsilon$ indicates a probability threshold, if the value $\varphi_{\mu,\sigma^2}(x) < \varepsilon$, it is considered as an

anomaly if $\varphi_{\mu,\sigma 2}(x) \geq \varepsilon$, it is considered as a normal data instance. The selection of value $\varepsilon$ depends on a specific domain, and several techniques are available for it.

Regression modeling has been extensively investigated for time series data. Firstly, a regression model is fitted to the data. Then, for each test instance, the residual for the test instance is used to determine the anomaly score. The statistical tests have been proposed to determine anomalies with a certain confidence.

In a mixture of parametric distribution modeling, it is first determined to which distribution training instances belong and then they are modeled separately for their parametric distribution. A test instance which does not belong to any of the learnt models is declared to be anomalous.

In a non-parametric statistical technique, few assumptions regarding the data are made, instead, the probability distribution is determined by the given data. There are two main methods, one is histogram based and the other one is kernel function based.

Histogram based anomaly detection is the simplest non-parametric statistical technique. The histogram is used to maintain a profile of the normal data. A frequency histogram based on the values in the training data is built. In the second step, the technique checks if a test instance falls into any one of the bins of the histogram. If it does, the test instance is normal, otherwise it is anomalous. A variant of the basic histogram based technique is to assign an anomaly score to each test instance based on the frequency of the bin in which it falls.

The other non-parametric statistical modeling is kernel function anomaly detection. Kernel function estimation is a fundamental data smoothing problem where inferences about the population are made, based on a finite data sample.

Assume $\{x^1, x^2 ... x^n\}$ is an independent and identically distributed sample drawn from some distribution with an unknown density $f$. Then the kernel function is chosen to fit with the sample, and then the kernel density estimator is:

$$f = \frac{1}{nh} \sum_{i=1}^{n} K\left(\frac{x-x_i}{h}\right) \qquad \text{(Eq. 6)}$$

Where K () is the kernel, which is a non-negative function that integrates into one and has mean zero; h is a non-negative smoothing parameter called the bandwidth. Intuitively, h should be as small as the data allows, however, there is always a trade-off between the bias of the estimator and its variance.

There are also classification based anomaly detection techniques. Classification is used to learn a model (classifier) from a set of labeled data instances (training) and then, a test instance is classified into one of the classes using the learnt model. There needs to be a precondition: a classifier that can distinguish between normal and anomalous class. Anomalies can be multi-class and one-class. In multi-class classification, several classes can be defined as normal. In respect of a test instance, getting through the learnt classifier, if it is prohibited to be included in any of the classes, the test instance is considered as anomalous. In one-class classification, test data can only be classified as normal or anomaly.

Neural networks, Bayesian networks, Support Vector Machines (SVMs) and Rule based techniques are four main techniques in classification based anomaly detection. In these techniques, neural networks and rule based can be applied to anomaly detection in multi-class as well as one-class setting; Bayesian networks have been used for anomaly detection in the multi-class setting; Support Vector Machines (SVMs) have been applied to anomaly detection in the one-class setting.

There are also clustering based anomaly detection techniques. Clustering is used to group similar data instances into clusters. There are mainly three categories: Firstly, if normal data instances and anomalies belong to different clusters, a known clustering based algorithm is applied to cluster the data set and excluded data instances are indicated as anomalies. Secondly, test data are clustered into different clusters. The data instances that lie close to a cluster centroid are considered normal, while those that lie far away from any cluster centroid are considered as anomalies. The distance to the closest cluster centroid is its anomaly score. Thirdly, normal data instances are considered as those that appear in large and dense clusters, while instances appearing in small and sparse clusters are labeled as anomalies.

There are also nearest neighbor based anomaly detection techniques. For situations where normal data instances gather in dense neighborhoods, while anomalies are quite far away from their closest neighbors, a nearest neighbor based technique is applied. There are two main approaches: computing the test data's distance with its $k^{th}$ nearest neighbor as a score and calculating the test data's relative density, i.e. the number of neighbors within a given distance, as an anomaly score.

As mentioned above, a Recurrent Plot is used in one implementation of the system. The Recurrence Plot was introduced in 1987 by J. -P. ECKMA to analyze physiological systems (J.P.Eckman, S.Oliffson Kamphorsta. "Recurrence Plots of Dynamical Systems", Europhys. Lett., 4 (91, pp. 973-977 (1987)), in particular non-linear and dynamic systems. The data $X$ that are analyzed can be represented as being in a d-dimensional state space. In that state space they describe an orbit. The recurrence plot is designed to recognize such orbits, by their nature to be periodic. $x$ is the $i^{th}$ point on X, for $i = 1, ... N$. The recurrence plot is a $N * N$ matrix of dots, where a dot is placed at $(i,j)$, whenever $x(j)$ is sufficiently close to *x(i)*. When there is periodicity, lines will appear in the plot, next to the obvious line along the diagonal.

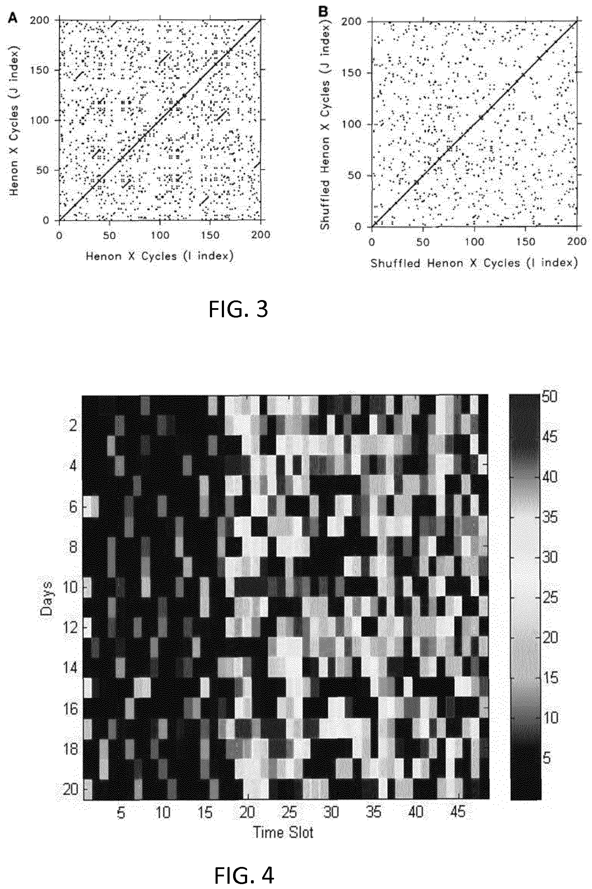

Figure 3 shows two examples of a recurrence plot.

There are several techniques which make use of the various analytical tools explained above. However, none of them can find a specific anomalous time stamp or timeslot and they are not insensitive to activity permutation. Since human activity never has a fixed pattern, an anomaly detection method should account for potential permutations in the current activity sequence. Also, we should make sure the found normal pattern is representative enough.

The system of the invention thus aims to find exact anomalous timeslots, so a more explicit reason for activity pattern deviation can be derived. People may change their activity schedule a little bit, without it being considered to be anomalous.

A typical system of the invention makes use of various sensors mounted in the house such as open/close sensors (for doors, windows, cupboard doors, fridge, washing machine etc.), passive infrared (PIR) sensors, humidity/temperature sensors. Also, wearable sensors carried by the user can be included, or solely used. An example is a pendant or wrist-worn device, for example providing immediate support to the user in case of emergency, that holds sensors like accelerometers, gyroscopes, magnetometers, and air pressure sensors. Also, heart rate, respiration, temperature, skin conductivity, blood pressure, sugar and other physiological sensors can be used.

These sensors are mounted in the kitchen, bedroom, bathroom and all places of one house, to monitor behavior in that house. For example, the presence in the rooms in the house, or detected movement times in rooms are taken as input signal.

In more detail, sensor examples are:

Kitchen: Open/close sensor on the refrigerator; Open/close sensor on cutlery draw/cabinet; Power sensor on water heater/coffee machine; PIR sensor placed in such a way that it has a clear side of the location where cooking takes place;

Living room: PIR sensor placed in such a way that it detects the area where the subject normally us in the living room; Pressure mat on the seat/couch normally used; Power sensor on TV;

Bathroom/Toilet: Pressure mat in the toilet; PIR sensor placed in such a way that it detects as much of the bathroom as possible; Temperature/humidity sensor which is not placed close to a wet area or ventilation;

Bedroom: Pressure mat in bed; PIR placed in such a way that it covers at least the bed;

Front door: Pressure mat; In/out home sensor

The analysis below is based on activity daily living data obtained from movement times as detected by the PIR motion sensors, mounted in several areas in a house.

The activity density map (ADM) is an effective way to view activity density. In an ADM, the sensor signals are structured in a matrix of daily patterns. The row indicates the day, and the column indicates the timeslot of a day. The value at a specific coordinate represents the density value (i.e. cumulative sensor data) at the specific time of that specific day. This can be done for all day, i.e. the rows hold contiguous days, but also for selected days, which are representative. For example, if the cleaning lady is present on Fridays, the signals from Fridays can be taken apart in a separate matrix.

Figure 4 shows an image of an activity density map, where 20 days are collected and a day is divided into 48 timeslots (the activity density is calculated every half hour). This particular example show activity levels as determined by an accelerometer worn by a user. The activity levels may relate to any of the sensor signals outlined above, either as raw signals or processed signals. For example a number of PIR sensor firing times, may be measured which means the activity density then reflects the amount of movement in the observation window. The density *N* can then be computed

$$N = \frac{s}{t}$$

as the number s of all motion sensor hits during a period time divided by that period duration t: where t=half an hour in this example. The ADM (Activity Density Map) may also hold activity types (sleeping, eating, bathing, watching TV, etc.). Different grey levels in Figure 4 represent different levels of density.

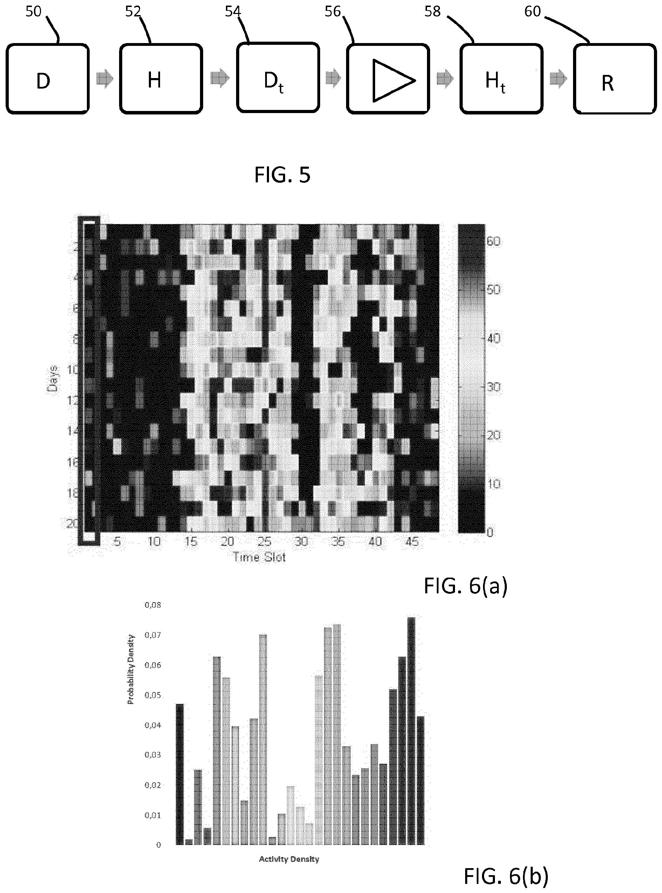

Figure 5 shows the reference day calculation process used for histogram based anomaly detection. The principle is to maintain a profile of a normal data by observing a set of days and the average pattern is taken from those previous days. The pattern is called the reference day. More precisely, in the reference day per timeslot a *b*-bin histogram is stored which captures the probability density distribution of the activity level at that timeslot.

A value $D_{d,t}$ is defined to represent the activity density on day *d* at timeslot *t*. For an ADM that has *N* days and T timeslots per day:

$$D = \left\{ D_{d,t} \mid d = (1,2,3,\ldots N)\, \& \, t = (1,2,3,\ldots T) \right\}$$

is defined as the ADM density set.

The values D are collected in step 50.

Based on all activity densities in $D$, a b-bin equal-size normalized Histogram $H$ is created in step 52 (for each bin, the number of values from the data set that fall into each bin are counted, and the percentage is calculated).

In an ADM, each timeslot's density $D_t = D(t \in (1,2,3,... T_n))$ is obtained in step 54 and then in step 56 compared with b bins of the histogram $H$ (histogram based on whole ADM densities). The number of values from the data set that fall into each bin is counted.

By normalizing, the probability distribution of timeslot t is obtained in step 58: $H_t = P(t)(t \in 1,2,...T)$. $H_t$ indicates the (reference) activity behavior at timeslot $t$.

This process is applied for all T timeslots, and the respective $H_t$ are stored in step 60 the reference day $R = \{H_1, H_2 ... H_T\}$. R is a $T$ joint probability distribution histogram.

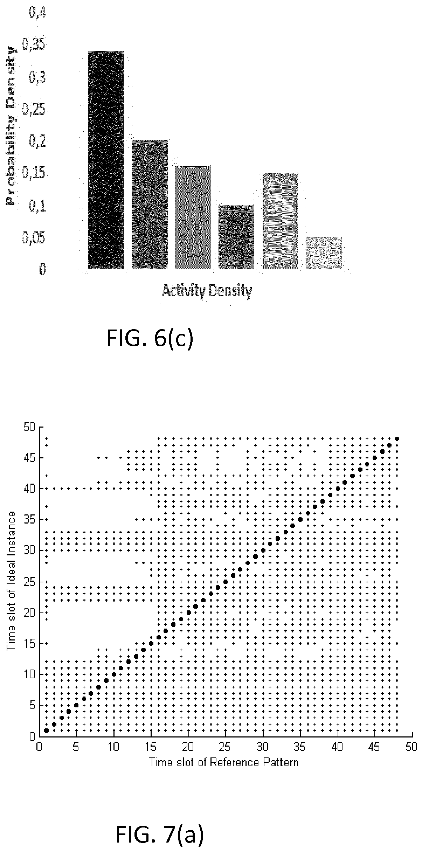

Figure 6 shows this example generation process for a normalized 30-bin ADM probability distribution histogram for timeslot t (the first timeslot in this example). Different grey levels mean different density.

Figure 6(a) shows the sensed ADM. Figure 6(b) shows the 30-bin ADM probability distribution histogram $H$ for all ADM densities. Figure 6(c) shows the extracted sensed ADM's for the first timeslot densities. By counting the number of values from the data set that fall into each bin of Figure 6(b), the probability histogram of Figure 6(c) is obtained as $H_t$.

In order to allow permutation of the order of activities, the concept of a Recurrence Plot for physiological systems as explained above is applied to the activity density maps. This plot is essentially a matrix of values.

In this case, a data sequence is not compared with itself, but a measured day's density data $M$ is compared with reference day R. $M$ is a 1-dimensional temporal sequences with $T$ elements, $m(i) \in M$ is defined as an activity density at timeslot i. R is a b-dimensional temporal sequence with T elements, $H_t$ is the $t^{th}$ element in R, it is a b-bin probability histogram $H_t = \{p_1, p_2, ...p_b\}$. When $m(i)$ is compared with $H_t$, the probability corresponding to the value $m(i)$ is stored at the element (t,i) in the recurrence plot. A simpler version of the recurrence matrix (plot) is obtained by quantizing the probability values to a binary outcome: if the probability $H_t$ of $m(i)$ is above a threshold $\gamma$, $p(H_t, m(i)) \geq \gamma$, it is considered as a recurrence point $P_r$ in recurrence plot, i.e. value is 1. Otherwise its value is quantized to zero (no dot in the plot).

$$P_r = \{(t,i)|p(H_t, m(i)) \geq \gamma\} \qquad \qquad \text{(Eq. 7)}$$

In the recurrence plot, all recurrence points $P_r$ are marked as 1, others as 0. This leads to a matrix of 0/1 values.

Figure 7(a) shows a recurrence plot, in which the x-axis represents the reference day timeslots R, and the y-axis represent the measurement day $M$, timeslots. The recurrence points are designated as the pixels dots. Recurrence points in the diagonal are designated as larger pixel dots. Note that the quantization is not essential. If no quantization is applied, a 3D representation would be needed.

A recurrent point in the diagonal means that for the particular timeslot, the actual activity density observed has a probability of arising in the reference day which exceeds a threshold probability. Thus, taking a row of the grid, the points indicate the timeslots in the reference pattern where the observed activity level could conceivably arise. If the observed activity level could not conceivably arise at the corresponding timeslot in the reference pattern (based on the histogram such as in Figure 6(c)) then this is an indication of a potential anomaly, in that the actual day is not close to the reference day.

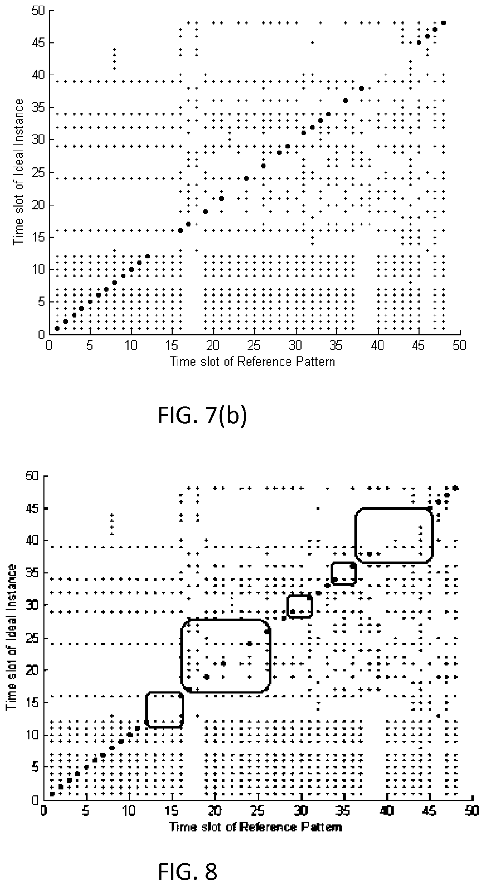

Missing larger pixel dots indicate a potential anomaly. Without anomalies, the diagonal line is fully filled with dots as shown in Figure 7(a). Figure 7(b) shows an incomplete diagonal line with potential anomaly.

If no quantization is applied, the sum of values along the diagonal could be computed, as example metric to decide whether the day is anomalous. A high value indicates normal, a low value indicates abnormal. This computation is similar to computing the correlation between the test and reference day. This test is less sensitive to an anomaly at a single timeslot, while all others are normal. The single anomaly will average out and might remain undetected. Therefore, a stronger (first) test is to test every timeslot along the diagonal. This basically returns the effect of quantization and testing for missing dots.

Thus, a recurrence plot describes the correlation between a measurement day and the reference day. On the diagonal, the correspondence between timeslots of the measured day with the same timeslot of the reference day appears, while off-axis the correspondence of the measured timeslot to another timeslot during the reference day appears. This information is used to search for permutations. The correlation is present in the recurrence plot as qualitative features by means of the isolated points along the diagonal, and by horizontal and vertical lines, and by bands of white space and so on as described in CHARLES L. WEBBER, JR., AND JOSEPH P. ZBILUT. "Dynamical assessment of physiological systems and states using recurrence plot", Journal of Applied Physiology 03/1994; 76(2):965-73.

The most important qualitative feature in the activity density recurrence plot is the missing recurrence points along the upward diagonal line $P_m$. In the unquantized version, these are the points along the diagonal with a low probability value. They indicate the timeslots where the measured day deviates from the reference. The other way around, the present recurrence points along the upward diagonal line indicate the timeslots that do correspond with the normal reference behavior. Hence, the number of points present is a measure for the "normality" of the measurement day.

$$P_m = \{(t,i)|p(H_t, m(i)) \leq T \& t = i\} \qquad \text{(Eq. 8)}$$

Figure 8 shows recurrence plot of Figure 7(b) with missing recurrence points in upward diagonal line. In the identified rectangular areas, there are missing recurrence points in the upward diagonal line.

In the next step of the algorithm, the missing points along the upward diagonal line $P_m$ are further evaluated as to whether they are anomaly timeslots indeed or that they could be permuted with another timeslot.

The missing recurrence points along the upward diagonal line $P_m$ declare inconformity of normal behavior. However, while some missing points $P_m$ are caused by real uncommon activities, others might in fact be originated by a permutation of the activities.

When this permutation happens within a reasonable time period, these missing points should not be counted as anomalous timeslots. Therefore, a test for permutations is used to filter out these situations.

A simple way to test whether the two sequences are a permutation of each other is by normalizing the sequences according to a ranking operation and to compare the equality of the resulting sequences element by element. In case of scalar sequences, the ranking can be linear. For example, numerical values can be ranked from small to large, or from large to small.

In a typical case, the measurement data in the activity density map are scalar, a single activity level, but the reference data in the reference map are vectors representing a probability distribution (histogram). The needed value from each histogram depends on the value of the (used) timeslot in the measurement sequence. To solve the permutation test, the underlying concept of the recurrence plot is used. The matrix of possible values is created and the probability sum along the diagonal is maximized by interchanging rows (or columns) in the matrix. Maximizing the probability sum is to be understood as finding as many as possible acceptable values along the diagonal. Acceptable means that at the corresponding slot a sufficient probability level is found. The sum of probabilities might be smaller if that would increase the total number of acceptable slots. It is clear from this reasoning that quantizing the probability values simplifies to performing this maximization objective.

Other forms of representing the measurement day and reference day can be envisioned. Also other maximization or test criteria can be envisioned. Both may lead to other forms of performing the permutation test.

The permutation test may use a recursive process or a backtracking process, and examples of each of these are given below.

The permutation test is applied to one missing point $p_m \in P_m$ at a time. A window centered around $p_m$ is chosen that defines the time span over which candidate permutations are evaluated. The window can be the full day. Preferably, however, it is chosen in accordance with the type of activity for that part of the day. For example, dressing, breakfast, and personal care happen in the morning, and the window may search for a permutations in the order during the morning part of the day. To simplify, a fixed window can be chosen, however of limited size.

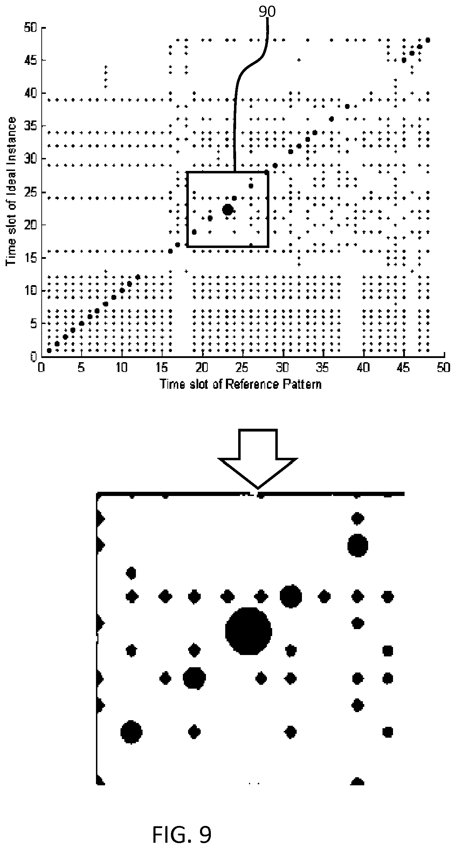

The test is for example executed in a recursive manner. As depicted in Figure 9, a square window 90 is centered around the current (missing) timeslot $p_m$. The window is square with an odd number of elements, so it extends symmetrically on both sides. A typical number is 11 slots (5 up and 5 down). At the boundaries of the recurrence plot the windows are clipped to that boundary. Another, and preferred, option is to cyclically repeat the recurrence plot at its boundaries such that at the boundaries the window can extend into those repetitions. This can be implemented by letting the window include the recurrence plot's values at the opposite boundary, e.g. by using a modulo operator on the index.

In the selected window 90, a row represents a timeslot of the measured day, and a column represents a timeslot of the reference day. Recurrence points indicate a match between the timeslots of the measurement day with the respective timeslots in the reference day. In the permutation test, the center point will be a missing point, by the way the window was selected. Along the row of this non-matching timeslot $t_m \in T_m$ the other timeslots are tested for a match. If another recurrence point $p_r = \{\{(t_r, t_m)|p(H_{t_r}, m(t_m)) \geq T\}\}$ is selected, the assumption is made that the activity performed at $t_m$ of the measured day happens at $t_r$ in the reference day. Based on this assumption, $t_m$ and $t_r$ can be swapped. Of course, while a swap may repair the current non-matching timeslot, it may cause the other timeslot (with whom is swapped) to become non-matching. So within the window, continually recursive searching is done for the following timeslots, and all swap possibilities need to be tested. When a full match is found, further testing can stop, and the current $t_m$ is marked as normal.

The search can be implemented in a recursive manner. Given a window of a certain size N, a row is chosen whose point at the diagonal is a missing one and for which point a candidate swap exists. A candidate swap is one in which the missing point gets replaced by a match. The row is evaluated for all candidate swaps. For each candidate, the two columns are swapped and, after the swap, the row and column of the repaired point are removed from the window. The resulting window, of size N-1, is submitted to the same routine, hence the recursion. The routine returns back the minimum obtainable number of missing points along the diagonal. For each candidate this is the number returned by the recursively called routine (on the sub-window). So, if one of the recursively called routines returns a zero the calling routine can stop for evaluation and return a zero. Note that if there exists no selected row (missing points on the diagonal and no candidate swaps), i.e. the whole window is empty, the routine returns the size of the window. As explained above, if the diagonal has no missing points, the value 0 is returned.

Some examples of this process will be explained in more detail. As mentioned above, this test is performed for all diagonal non-matching timeslots $T_m$. If the function yields the minimum obtainable number $N_0$, in this algorithm the threshold would be zero $Thr_{N_0} = 0$, which means a full match. If $N_0 = 0$, calculation stops, which means $t_m$ is normal. If the value $N_0 > 0$, the function will go back to the last layer, restore the last bigger matrix and try other recurrence plots until all the possible situations are tried. Finally, an alert is raised if all the possible situations are tried, the obtained minimum number $N_0$ of non-matches exceeds threshold $Thr_{N_0} = 0$.

For ease of explanation, binary values are assumed for the activity level or activity type.

A first example of an initial 7 x 7 matrix is shown below.

**Table 1**

|   | A | B | C | D | E | F | G |
|---|---|---|---|---|---|---|---|
| 1 | 1 | 1 | 1 | 1 | 1 | 1 | 1 |
| 2 | 1 | 0 | 0 | 1 | 0 | 1 | 1 |
| 3 | 1 | 1 | 1 | 1 | 1 | 1 | 1 |
| 4 | 1 | 0 | 0 | 0 | 0 | 1 | 0 |
| 5 | 1 | 1 | 1 | 1 | 1 | 1 | 1 |
| 6 | 1 | 0 | 0 | 1 | 1 | 0 | 1 |
| 7 | 1 | 1 | 0 | 0 | 1 | 1 | 1 |

In this example, there are two zeros on the diagonal (bottom left to top right) at 6B and 4D giving two initial anomalies.

The algorithm then searches for permutations that minimize the number of zeros on the diagonal.

Starting with the center element (4th row, column D: 4D), there are two candidates for a swap: 4A and 4F.

The table below shows an attempted swap with 4A so that columns A and D are swapped.

**Table 2**

|   | D | B | C | A | E | F | G |
|---|---|---|---|---|---|---|---|
| 1 | 1 | 1 | 1 | 1 | 1 | 1 | 1 |
| 2 | 1 | 0 | 0 | 1 | 0 | 1 | 1 |
| 3 | 1 | 1 | 1 | 1 | 1 | 1 | 1 |
| 4 | 0 | 0 | 0 | 1 | 0 | 1 | 0 |
| 5 | 1 | 1 | 1 | 1 | 1 | 1 | 1 |
| 6 | 1 | 0 | 0 | 1 | 1 | 0 | 1 |
| 7 | 0 | 1 | 0 | 1 | 1 | 1 | 1 |

After this swap there are still two zeros on the diagonal. The zero at 4D has been repaired (it is now labeled 4A) but a zero at 7A (it is now labeled 7D) has returned.

The other candidate 4F is tried, Columns D and F are of the original Table 1 are swapped:

**Table 3**

|   | A | B | C | F | E | D | G |
|---|---|---|---|---|---|---|---|
| 1 | 1 | 1 | 1 | 1 | 1 | 1 | 1 |
| 2 | 1 | 0 | 0 | 1 | 0 | 1 | 1 |

(continued)

|   | A | B | C | F | E | D | G |
|---|---|---|---|---|---|---|---|
| 3 | 1 | 1 | 1 | 1 | 1 | 1 | 1 |
| 4 | 1 | 0 | 0 | 1 | 0 | 0 | 0 |
| 5 | 1 | 1 | 1 | 1 | 1 | 1 | 1 |
| 6 | 1 | 0 | 0 | 0 | 1 | 1 | 1 |
| 7 | 1 | 1 | 0 | 1 | 1 | 0 | 1 |

There is now one zero on the diagonal. The zero previously at 4D has been repaired, and no other zero has returned.

Thus, there is an improvement.

The same routine is called again to find out whether further improvement is achievable based on the starting point of having already swapped columns D and F.

The fourth row and fourth column (labeled 4 and F above) are first removed, since they no longer need to be considered:

**Table 4**

|   | A | B | C | E | D | G |
|---|---|---|---|---|---|---|
| 1 | 1 | 1 | 1 | 1 | 1 | 1 |
| 2 | 1 | 0 | 0 | 0 | 1 | 1 |
| 3 | 1 | 1 | 1 | 1 | 1 | 1 |
| 5 | 1 | 1 | 1 | 1 | 1 | 1 |
| 6 | 1 | 0 | 0 | 1 | 1 | 1 |
| 7 | 1 | 1 | 0 | 1 | 0 | 1 |

This matrix then serves as matrix for the first step above, however with a matrix of size one less. Thus, it is used as the initial matrix for the second cycle of the recursive process.

As will have been seen, for simplicity and to enable the column movements to be seen more easily, the row and column names are not changed to reflect the shrink to a 6 x 6 matrix. In the algorithm, the columns will in practice be renamed.

There is one zero on the diagonal at 6B.

The algorithm searches for a permutation that minimizes number of zeros on diagonal. Starting with the element 6B (the only zero on the diagonal in this example). In row 6 there are four candidates: 6A, 6E, 6D, 6G. The selection is for example ordered alphabetically in the recursive process, so that the first attempt for a permutation is using 6A.

Columns A and B are swapped:

**Table 5**

|   | B | A | C | E | D | G |
|---|---|---|---|---|---|---|
| 1 | 1 | 1 | 1 | 1 | 1 | 1 |
| 2 | 0 | 1 | 0 | 0 | 1 | 1 |
| 3 | 1 | 1 | 1 | 1 | 1 | 1 |
| 5 | 1 | 1 | 1 | 1 | 1 | 1 |
| 6 | 0 | 1 | 0 | 1 | 1 | 1 |
| 7 | 1 | 1 | 0 | 1 | 0 | 1 |

There are now no zeros on the diagonal. The zero previously at 6B has been repaired, and no other zero has returned.

14

In this example, a minimal score has been achieved and the routine ends, returning a zero to the caller of the routine.

The caller then receives a notification of zero anomalies from the recursively called subroutine. In this example, it is concluded that the anomalies at the diagonal can be repaired by a permutation.

The algorithm then proceeds to the other initial anomalies until all have been tested, and a total score can be returned.

A second example of an initial 7 x 7 matrix is shown below:

**Table 6**

|   | A | B | C | D | E | F | G |
|---|---|---|---|---|---|---|---|
| 1 | 1 | 0 | 1 | 1 | 1 | 1 | 1 |
| 2 | 1 | 0 | 0 | 0 | 0 | 1 | 1 |
| 3 | 1 | 1 | 1 | 1 | 1 | 1 | 1 |
| 4 | 0 | 0 | 0 | 0 | 0 | 1 | 1 |
| 5 | 1 | 1 | 1 | 1 | 1 | 1 | 1 |
| 6 | 0 | 0 | 0 | 1 | 0 | 0 | 0 |
| 7 | 1 | 1 | 0 | 0 | 1 | 1 | 1 |

There are two initial anomalies on the diagonal. The algorithm searches for permutations that minimize number of zeros on the diagonal.

Starting with the center element: 4D. On row 4 there are two candidates: 4F and 4G.

Trying 4F involves swapping columns D and F:

**Table 7**

|   | A | B | C | F | E | D | G |
|---|---|---|---|---|---|---|---|
| 1 | 1 | 0 | 1 | 1 | 1 | 1 | 1 |
| 2 | 1 | 0 | 0 | 1 | 0 | 0 | 1 |
| 3 | 1 | 1 | 1 | 1 | 1 | 1 | 1 |
| 4 | 0 | 0 | 0 | 1 | 0 | 0 | 1 |
| 5 | 1 | 1 | 1 | 1 | 1 | 1 | 1 |
| 6 | 0 | 0 | 0 | 0 | 0 | 1 | 0 |
| 7 | 1 | 1 | 0 | 1 | 1 | 0 | 1 |

There are still two zeros on the diagonal.

The zero at original position 4D has been repaired (now a 1 at 4F), however a zero at original position 2F (now 2D) has returned. There is no improvement so another candidate is tried. Trying 4G involves swapping columns D and G of the initial matrix:

**Table 8**

|   | A | B | C | G | E | F | D |
|---|---|---|---|---|---|---|---|
| 1 | 1 | 0 | 1 | 1 | 1 | 1 | 1 |
| 2 | 1 | 0 | 0 | 1 | 0 | 1 | 0 |
| 3 | 1 | 1 | 1 | 1 | 1 | 1 | 1 |
| 4 | 0 | 0 | 0 | 1 | 0 | 1 | 0 |
| 5 | 1 | 1 | 1 | 1 | 1 | 1 | 1 |
| 6 | 0 | 0 | 0 | 0 | 0 | 0 | 1 |

(continued)

|   | A | B | C | G | E | F | D |
|---|---|---|---|---|---|---|---|
| 7 | 1 | 1 | 0 | 1 | 1 | 1 | 0 |

There is now one zero on the diagonal. The zero at original position 4D (4G in the new table) has been repaired, and no other zero has returned. There is an improvement so the same routine is called recursively to find out whether further improvement is achievable (for this candidate swap D-G).

A sub-matrix is created after removing the fourth row and fourth column (labeled G) from the previous table:

**Table 9**

|   | A | B | C | E | F | D |
|---|---|---|---|---|---|---|
| 1 | 1 | 0 | 1 | 1 | 1 | 1 |
| 2 | 1 | 0 | 0 | 0 | 1 | 0 |
| 3 | 1 | 1 | 1 | 1 | 1 | 1 |
| 5 | 1 | 1 | 1 | 1 | 1 | 1 |
| 6 | 0 | 0 | 0 | 0 | 0 | 1 |
| 7 | 1 | 1 | 0 | 1 | 1 | 0 |

This matrix serves as the initial matrix for the first step, however with a matrix of size one less.

There is one zero on the diagonal at 6B. The algorithm searches for a permutation that minimizes number of zeros on the diagonal. Starting with element 6B (the only zero on the diagonal in this example) there is one candidate at location 6D. Thus, columns B and D are swapped:

**Table 10**

|   | A | D | C | E | F | B |
|---|---|---|---|---|---|---|
| 1 | 1 | 1 | 1 | 1 | 1 | 0 |
| 2 | 1 | 0 | 0 | 0 | 1 | 0 |
| 3 | 1 | 1 | 1 | 1 | 1 | 1 |
| 5 | 1 | 1 | 1 | 1 | 1 | 1 |
| 6 | 0 | 1 | 0 | 0 | 0 | 0 |
| 7 | 1 | 0 | 0 | 1 | 1 | 1 |

There is still one zero on the diagonal. The zero at previous position 6B (6D in figure) has been repaired, however a zero at previous 1D (now 1B) has returned.

There are no other candidates. The minimal score has been achieved and the routine ends, returning a 1 as the minimal number of zeros on the diagonal to its caller. The caller thus receives 1 anomaly from the recursively called subroutine.

In this example it is concluded that the anomalies at the diagonal can be repaired by a permutation only up to 1 remaining.

In case there was a third candidate at row 4 (of the initial routine), that candidate would be evaluated in a similar manner. When the resulting number of anomalies would be less, that lesser number would be returned.

In these examples there is one nesting level of recursion. It is clear of course that further recursion can happen.

Also it is worth noting that in this example, at each level of recursion a single row is evaluated for example the one at the center, or closest to the center (in case of even number of rows). In the second example, for example, in Table 10 the anomaly at 1B (as labeled in that Table) could be tested for permutations. Position 1A would be a candidate and after swapping, zero anomalies would result. This type of swapping, i.e. this type of permutation, may also be allowed.

This would provide another design to evaluate for permutations. Note that this type of test could be implemented in a different way, not using recursion. For example, the search could be a back-tracking algorithm, in which to every column a different row is assigned, the chosen row to be out of the set of (not yet chosen) rows and having a 1 in the current column.

For example, a backtracking approach will be explained with reference to a 5x5 matrix:

**Table 12**

|   | A | B | C | D | E |
|---|---|---|---|---|---|
| 1 | 1 | 0 | 1 | 1 | 1 |
| 2 | 1 | 0 | 0 | 1 | 1 |
| 3 | 0 | 1 | 1 | 0 | 1 |
| 4 | 1 | 0 | 1 | 1 | 0 |
| 5 | 0 | 1 | 1 | 0 | 0 |

The first step is to:

1. Assign row 1 to column A so that it becomes the lowest row so that the cell at 1A end up on the diagonal, at position 5A
2. Assign row 3 to column B
3. Assign row 4 to column C
4. Assign row 2 to column D
5. Row E cannot be assigned (row 5 remains, but it has a zero in column E).
A 1 anomaly solution is stored in memory as a minimum.
Backtracking to step 4 (and memorizing that row 2 cannot again be assigned to column D)
6. Row D cannot be assigned (rows 2 and 5 remain: 2 has been tried, 5 has a zero in column D)
Backtrack to step 3 (and memorizing row 4 cannot again be assigned to column C]
7. Assign row 5 to column C
8. Assign row 4 to column D
9. Assign row 2 to column E

In this case, all columns can be assigned, so that a permutation exists that fully maps the reference with zero anomalies.

All of these approaches involve performing a test of activity permutations to find timeslots of the activity density map which are interchangeable to remove as many of the initial anomaly points as possible, and identifying the remaining anomaly points as a first anomaly indication.

Also, these approaches all involve setting a time window centered on an initial anomaly and testing for swaps of timeslots within the time window which remove the initial anomaly. It is also determined whether or not the timeslot swaps create new anomalies to see if the candidate swap is worth proceeding with.

There are many different permutation approaches to achieve the general aim of testing to see if timeslot variations render the activity density map compliant with the reference map.

In an actual software implementation, some extra steps are added to speed up the computation. For example, by testing for empty vertical and horizontal lines, it can rapidly be concluded that no match is possible.

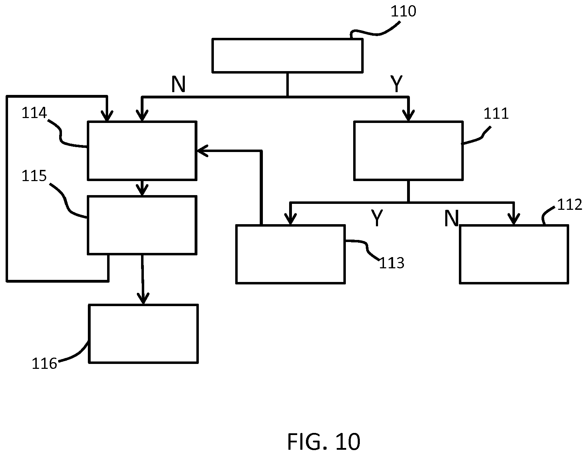

Figure 10 shows a permutation test process using simple calculations. It is applied to a window of data, such as shown in the tables above.

In step 110, a first check is made if there are empty (i.e. full of zeros) horizontal or vertical lines in the map.

An empty horizontal line means the activity (activity level or type) at the timeslot in the activity density map does not match any timeslot in the reference map so no swap can resolve the issue.

An empty vertical line means the activity (activity level or type) at the timeslot in the reference map is not recognized over the whole of the time period covered by the window of data. Again, for the permutation routine, there is no point to try candidate timeslots that fill the zero at the diagonal, since the corresponding swap will create a zero at another diagonal entry.

This holds for the case where we the entries are quantized to 0 and 1. In a scheme where the actual probabilities are used, the zeros are low probabilities and a swap may still yield a more advantageous permutation. This depends on the evaluation criteria.

For example, the criteria may not only require as many as possible dots along the diagonal, but may also require a certain minimum sum of probabilities, such that an additional missing dot might in the end provide a better sum - i.e. a more likely overall match. In this type of refinement the empty row and empty column should not be used to shortcut the evaluation, at least not in the described manner.

If there are empty lines, a test is made in step 111 for other missing diagonal points. If there are no other missing diagonal points, the process is finished in step 112 and the empty timeslot is identified as an anomaly. Thus a more rapid conclusion is reached that the initial anomaly is indeed to be reported.

If there are other missing diagonal points, the window size is reduced in step 113 until there is only one missing diagonal point. The routine then returns to the more complete process as described above, which commences at step 114, described below.

If in step 110 there are no missing lines identified, so the more complete process is to be followed as mentioned above, a recurrence point is selected (e.g. randomly) in step 114 to be used for the permutation test.

In step 115 a successful swap has been made in the manner as explained above, and the row and column is taken out to generate a smaller matrix.

Steps 114 and 115 are carried out recursively as shown so that for all candidate swaps, the the minimum number of remaining anomalies is returned in step 116.

The method involves evaluating all missing points along the diagonal of the original recurrence plot.

When moving the window along the diagonal of the recurrence plot, a permutation may be used to repair a missing dot (typically in the center of the window). The permutation as explained above achieves this aim, but, by virtue of the window size, neglects the further evaluation of the diagonal. This can be incorporated, however. For example, the routine may also return what permutations it is using in repairing the missing dot, and proceed along the diagonal, accounting for that permutation.

Further refinements, then, are to keep past columns fixed (in case they enter a next permutation window); to evaluate the diagonal from top to bottom; to use a window covering the full-day; to choose the window such that it includes all missing dots that would otherwise have overlapping windows; etc.

The description above relates to some specific examples and based on binary representations. Other representation and criteria may be used. The principles are clear from the description above, and can be generalized by those skilled in the art. The method is explained above using binary values for ease of explanation.

To further improve the anomaly diagnosis, other factors can be taken into account. Because of the character of the activity anomaly, only considering the correspondence of a single timeslot might not be sufficient. For example, if people stay in a quite low activity density during the whole day, it is quite possible that low activity appears at other timeslots, and the permutations on the recurrence plot prohibit to detect these anomalies, because these low densities may appear at every timeslot, which leads no missing diagonal point in the recurrence plot.

For this reason the permutation window is set to a maximum size. In this example the average activity density over the day might be too low compared to that of a normal day. Therefore, an additional test could be incorporated that evaluates the average activity density over the day. This additional metric can be included in the evaluation of the recurrence plot, so that together a more accurate detection can be realized.

For an extremely high or low activity density, which may have quite low probability in all timeslots of reference day, these activity densities can be identified as uncommon activity density.

$$T_u = \{i \, | \, p(H_t, m(i)) < T \& t \in \ (1,2, \dots 48) \, \} \qquad\qquad (\text{Eq. 9})$$

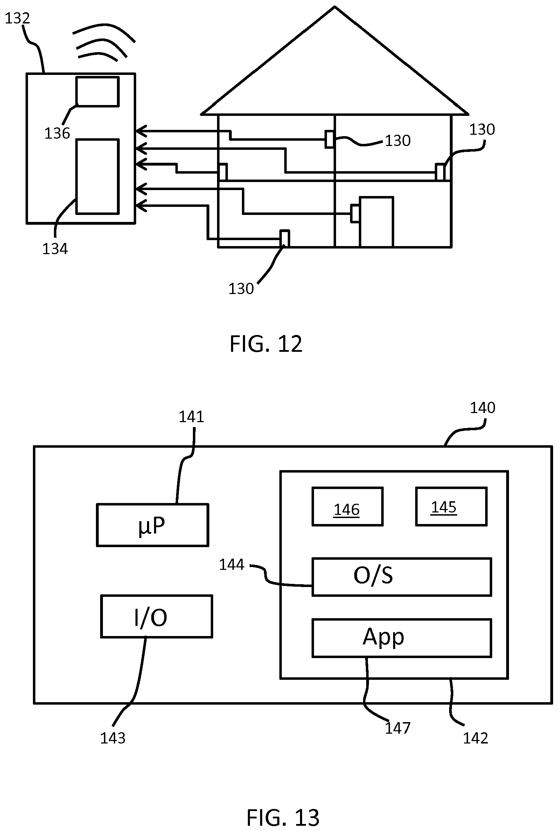

Uncommon activity density is an excessive situation, and will appear as an empty line in the recurrence plot. For example, in Figure 12 there are no recurrence points in the 10th row, and, as a result, the 10th timeslot of the measured day is marked as uncommon activity density. For this feature, a binary output can be used, if it is an uncommon activity density, the output is 1; otherwise the output is 0.

Another example metric that can be taken into account as a supplement is the day density variance $V_d$. Normally, we think a person's activity amount should vary in a reasonable range $[\mu - \sigma, \mu + \sigma]$. The reasonable range is generated from the whole ADM. Firstly, each day's average activity density is calculated. If an ADM has N days:

$$d_n = \sum_{i=1}^{T_n} D_n(i)/T_n \ (\, n \in N) \qquad\qquad (\text{Eq. 10})$$

Where $T_n$ is the number of timeslots, $n \in N$ is the number of days, $D_n(i)$ is the activity density at timeslot i in n-th day, $d_n$ is n-th day's average density value. After first step, we get an average day's density set $D_m = \{d_1, d_2, \dots d_N\}$. From this an average value for the day density $\mu$, and its standard deviation are derived:

$$\mu = \sum_{j=1}^{N} d_j /N \qquad\qquad (\text{Eq. 11})$$

$$\sigma = \sqrt{\frac{1}{N} \Sigma_{j=1}^{N} (d_j - \mu)^2} \qquad \text{(Eq. 12)}$$

Finally, when a day M is tested, its whole day average value is:

$$\bar{M} = \Sigma_{i=1}^{T_n} m(i) / T_n \ ( n \in N) \qquad \text{(Eq. 13)}$$

Where $m(i)$ is activity density timeslot $i$, $T_n$ Is number of timeslots. $\bar{M}$ is compared with $[\mu - \sigma, \mu + \sigma]$, and $D$ is its distance to the reasonable range:

$$V_d = (\bar{M} - \mu) / \sigma \qquad \text{(Eq. 14)}$$

If $V_d \le 2$, $M$ is considered as normal from the view of day density variance; if $V_d > 2$, $M$ is considered as an anomalous day density variance.

The description above shows that there is the computation of three features $f = \{f_1, f_2, f_3\}$, of which the method of the recurrence plot is the most comprehensive, each timeslot has a feature vector with three features, they are successively: permutation test score, uncommon activity density and day density variance. All three feature values are outputted as binary values 0/1. The output is 1, if the feature is considered as an anomaly for that timeslot, otherwise, the output becomes 0. For example, if a timeslot's feature is {1, 1, 0}, it means the timeslot is anomalous in the permutation test, has an uncommon activity density, but that day's average density variance is normal.

In the anomaly analysis, the three features are combined to yield an overall decision whether or not to raise an alert. To each timeslot, one out of four anomaly levels is assigned: normal, medium normal, medium anomaly, anomaly. The three features act equally in this classification. The anomaly level is computed as the sum over the feature set: 0, 1, 2 and 3. If $f = \{0, 0, 0\}$, all of the features indicate normal, the anomaly level is 0, the timeslot is considered as normal; If in $f$ one of the features indicate anomaly e.g. $f = \{0, 1, 0\}$, the anomaly level is 1 and the timeslot is considered as medium normal; If in $f$ two of the features indicate anomaly, the anomaly level is 2 and the timeslot is considered as medium anomalous; If in $f$ all of the features indicate anomaly, the anomaly level is 3 and the timeslot is considered as an anomaly.

This is only one way to combine metrics. Indeed, the concept underlying the invention relates to the first metric. Different methods can be combined to further improve the anomaly detection. For a vector feature, the decision may be based on a majority vote, logistic regression, Bayes classifiers, etc.

There are several parameters that may have an effect on the outcome of the algorithm, The following parameters are of interest:

(1) $N_b$ - Number of bins constituting the Histogram of the reference day

(2) $N_d$ - Number of days to be used in computing the reference day

(3) $N_t$ - Number of timeslots to represent a day

(4) $S$ - The size of the permutation window

(1) The size of the bin when building the histogram is quite important. If the bins are small, each bin is populated with a few training data, implying a high variance in the estimated probability to encounter that bin. Consequently, detection accuracy will be low or, when ignoring that variance, a high false alarm rate may be resulted. If the bins are large, there is little resolution also implying a low detection accuracy in particular missing anomalies at the boundary, i.e. resulting in a high false negative rate. In order to study $N_b$ the value of other parameters needs to be fixed. It has been found that a range 25 to 35 is found to be an optimal histogram bin number, which is used in the remaining evaluations.

(2) The number of Days in the Reference $N_d$ needs to give a sufficient collection time to obtain an accurate estimate of the activity distribution of the reference day. Longer collection time will improve the estimate, as long as the data can be considered stationary. On the other hand, unnecessarily long collection time will increase computational expense. Moreover, in practical applications, a long time will also imply a longer duration before the system is ready for (stable) usage. This also holds for the convergence time. It has been found that a number of collection days in the range 15 to 25 is found to be optimal.

(3) The number of timeslots for an optimal distinction of activities in an ADM is found to be between 30 and 60.

(4) For the window size *S*, in the permutation test, a square window with an odd number of elements is selected. The selected window size determines the time span over which permutations may happen. As activities are stored in timeslots of half an hour, indicating the window size by *S*, then the permutation span time is $\frac{S}{2} * 0.5 \text{ hour.}$ A window size of 5 to 15 slots is found to be effective.

The system and method is applicable for ADL sensors mounted in the home for monitoring the elderly behavior unobtrusively. The system is monitored by the caregiver, and the system gives the anomaly detection results to the caregiver, and the caregiver will decide if they will provide assistance.

The system is implemented as an algorithm to detect anomalies in the daily activities of elderly people. A particular aspect of the algorithm is that it accounts for possible permutations in the activities. The analysis through the anomaly detection provides an automated method for detecting specific anomalous timeslot of residents. Such detection will aid caregivers in the monitoring process, by alerting them so they can find out what exactly the problem is.

The method cannot only detect consecutive anomalies, but also quite single anomalous timeslots. The vertical axis represents 10 anomalous days, and the horizontal axis represent 48 timeslots of each day. The small points show detected anomalies, and the large circles indicate which of these are real.

Compared with existing approaches, a feasible way to find out specific anomalous timeslots is provided, while ignoring the influence of activity permutation in a suitable time window.

The permutation window size can be selected as desired. The selected ADM can be a fixed baseline period or a sliding baseline.

The behavior pattern of a user is learned from objective frequency histograms, instead of a static configuration based on inquiries or personal interviews. Also, the histogram based statistical techniques provide an unsupervised and justifiable solution for normal pattern construction. The approach using the whole house ADM provides personalized pattern of the resident, and little dependence is found on the actual floor plan.

This analysis results above relate only to ADM created from PIR data. The PIR motion sensor cannot identify specific individuals. Thus, the system will contain a degree of ambiguity as to who performed the activity (e.g., resident or visitor), and it is also a challenge to identify the number of persons in an apartment.

The system can be extended to multiple sensor data analysis for more detailed activity classification. The problem of pets could for example be solved with a special RFID tag on a pet's collar in the future.

Since the anomalous days in the test set consist dominantly of those having deviating activities, for example during the night, it is intuitive that a test of variance alone will be effective in detecting those days.

The recurrence plot used above may be replaced by the probability matrix. A measurement day can for example not only be compared with the reference day, but can also be compared with itself. The measurement-measurement recurrence plot also contains useful information.

Figure 13 shows the system. It comprises a set of sensors 130, of the types outlined above, positioned around the home. The sensor signals are provided to a central unit 132 which has a controller 134 for implementing the algorithms described above, and an output device 136. The output device 136 for example comprises a wireless transmitter for enabling remote access to the data by a caregiver, for example over the internet. The central unit may also have a screen to enable data to be presented locally.

Figure 14 illustrates an example of a computer 140 for implementing controller described above.

The computer 140 includes, but is not limited to, PCs, workstations, laptops, PDAs, palm devices, servers, storages, and the like. Generally, in terms of hardware architecture, the computer 140 may include one or more processors 141, memory 142, and one or more I/O devices 143 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 141 is a hardware device for executing software that can be stored in the memory 142. The processor 141 can be virtually any custom made or commercially available processor, a central processing unit (CPU), a digital signal processor (DSP), or an auxiliary processor among several processors associated with the computer 140, and the processor 141 may be a semiconductor based microprocessor (in the form of a microchip) or a microprocessor.

The memory 142 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 142 may incorporate electronic, magnetic, optical, and/or other types of storage media. Note that the memory 142 can have a distributed architecture, where various components are situated remote from one another, but can be accessed by the processor 141.

The software in the memory 142 may include one or more separate programs, each of which comprises an ordered listing of executable instructions for implementing logical functions. The software in the memory 142 includes a suitable operating system (O/S) 144, compiler 145, source code 146, and one or more applications 147 in accordance with exemplary embodiments.

The application 147 comprises numerous functional components such as computational units, logic, functional units, processes, operations, virtual entities, and/or modules.

The operating system 144 controls the execution of computer programs, and provides scheduling, input-output control, file and data management, memory management, and communication control and related services.

Application 147 may be a source program, executable program (object code), script, or any other entity comprising a set of instructions to be performed. When a source program, then the program is usually translated via a compiler (such as the compiler 145), assembler, interpreter, or the like, which may or may not be included within the memory 142, so as to operate properly in connection with the operating system 144. Furthermore, the application 147 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 143 may include input devices such as, for example but not limited to, a mouse, keyboard, scanner, microphone, camera, etc. Furthermore, the I/O devices 147 may also include output devices, for example but not limited to a printer, display, etc. Finally, the I/O devices 143 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 143 also include components for communicating over various networks, such as the Internet or intranet.

When the computer 140 is in operation, the processor 141 is configured to execute software stored within the memory 142, to communicate data to and from the memory 142, and to generally control operations of the computer 140 pursuant to the software. The application 147 and the operating system 144 are read, in whole or in part, by the processor 141, perhaps buffered within the processor 141, and then executed.

When the application 147 is implemented in software it should be noted that the application 147 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1.  An activity of daily living, ADL, monitoring system for monitoring ADLs of a person within an environment, wherein the ADL monitoring system comprises:

    a set of sensors (130) each adapted to respond to an activity and to generate a sensor output signal representative of the activity;
    a data processing unit (134) adapted to receive the sensor output signals and to process the sensor output signals, to:

    generate (10) an activity density map which identifies the level or type of a particular activity within particular timeslots;

generate (12) a reference map which indicates a reference value or range of values of activity levels or types within the particular timeslots;

compare (14) the level or type of a particular activity in the individual timeslots of the activity density map with the reference spread of activity levels or types in the corresponding timeslots of the reference map;

determine a size of correspondence of the level or type of activity arising in each timeslot of the activity density map with the reference spread of activity levels or types in the corresponding timeslots of the reference map to identify initial anomaly points;

**characterised in that** the data processing unit is further configured to:

for the initial anomaly points, perform a test of activity permutations to find timeslots of the activity density map which may be reordered to remove as many of the initial anomaly points as possible; and identify the remaining anomaly points as a first anomaly indication.

2. A system as claimed in claim 1, wherein the data processing unit (134) is adapted to perform the test of activity permutations by:

setting a time window centered on an initial anomaly;
testing for reordering of timeslots within the time window which remove the initial anomaly;
determining whether or not the timeslot reordering creates new anomalies.

3. A system as claimed in claim 2, wherein the data processing unit (134) is adapted to perform the test of activity permutations by recursively testing timeslot swaps within the time window to find the minimum remaining number of anomaly points for the time window.

4. A system as claimed in any preceding claim, wherein the activity density map and the reference map correspond to a time period of a set of complete days.

5. A system as claimed in any preceding claim, wherein the data processing unit (134) is adapted to determine the size of correspondence by determining a probability value of the activity level arising in each timeslot of the activity density map based on the reference map, and is adapted to optimize the total probability.

6. A system as claimed in any preceding claim, wherein the data processing unit (134) is adapted to generate the reference map as a sequence of activity probability distributions for each timeslot.

7. A system as claimed in claim 6, wherein the data processing unit (134) is adapted to:

form a recurrence plot from the sequence of activity probability distributions; and
identify the initial anomaly points as missing points from the main diagonal of the recurrence plot.

8. A system as claimed in any preceding claim, wherein the data processing unit (134) is adapted to:

identify timeslots which throughout the activity density map correspond to initial anomaly points, and provide a second anomaly indication based on the identified timeslots; and
obtain an average activity density for the activity density map, and compare the average activity density with the average activity density for the reference map, and provide a third anomaly indication based on the comparison.

9. A system as claimed in any preceding claim, wherein the set of sensors (130) comprise one or more of: PIR sensors; open/close sensors; power sensors; mat pressure sensors; radar and ultra-sound based sensors; humidity sensors; $CO_2$ sensors; temperature sensors; microphones; cameras; wearable sensors; accelerometers; gyroscopes; heart-rate monitors; respiration sensors; body temperature sensors; skin conductivity sensors; blood pressure sensors; sugar level detectors.

10. A computer-implemented method of monitoring activities of daily living, ADLs, of a person within an environment, comprising:

by means of a data processing unit (134),
receiving sensor output signals from a set of sensors (130) each adapted to respond to an activity and to generate a sensor output signal representative of the detected activity;
and processing the sensor output signals, to:

generate (10) an activity density map which identifies the level or type of a particular activity within particular timeslots;

generate (12) a reference map which indicates a reference value or range of values of activity levels or types within the particular timeslots;

compare (14) the level or type of a particular activity in the individual timeslots of the activity density map with the reference spread of activity levels or types in the corresponding timeslots of the reference map;

determine a size of correspondence of the level or type of activity arising in each timeslot of the activity density map with the reference spread of activity levels or types in the corresponding timeslots of the reference map to identify initial anomaly points; **characterised in that** the processing further comprises:

for the initial anomaly points, perform a test of activity permutations to find timeslots of the activity density map which may be reordered to remove as many of the initial anomaly points as possible; and

identify the remaining anomaly points as a first anomaly indication.

11. A method as claimed in claim 10, comprising performing the test of activity permutations by:

setting a time window centered on an initial anomaly;
testing for reordering of timeslots within the time window which remove the initial anomaly;
determining whether or not the timeslot reordering creates new anomalies.

12. A method as claimed in claim 11, comprising performing the test of activity permutations by recursively testing timeslot swaps within the time window to find the minimum remaining number of anomaly points for the time window.

13. A method as claimed in any one of claims 10 to 12 comprising determining the size of correspondence by determining a probability value of the activity level arising in each timeslot of the activity density map based on the reference map, and optimize the total probability.

14. A method as claimed in any one of claims 10 to 13, comprising:

generating the reference map as a sequence of activity probability distributions for each timeslot;
forming a recurrence plot from the sequence of activity probability distributions; and
identifying the initial anomaly points as missing points from the main diagonal of the recurrence plot.

15. A computer program comprising software code which is adapted to cause the system according to claim 1 to perform the method according to any one of claims 10 to 14, when executed on the data processing unit of said system.

**Patentansprüche**

1. System zur Überwachung einer Aktivität des täglichen Lebens, ADL (Activity of Daily Living), zum Überwachen von ADLs einer Person innerhalb einer Umgebung, wobei das ADL-Überwachungssystem umfasst:

einen Satz von Sensoren (130), die jeweils ausgebildet sind, auf eine Aktivität anzusprechen und ein Sensorausgangssignal zu erzeugen, das für die Aktivität repräsentativ ist;
eine Datenverarbeitungseinheit (134), die ausgebildet ist, die Sensorausgangssignale zu empfangen und die Sensorausgangssignale zu verarbeiten zum:

Erzeugen (10) einer Aktivitätsdichtekarte, die das Niveau oder die Art einer bestimmten Aktivität innerhalb bestimmter Zeitschlitze identifiziert;

Erzeugen (12) einer Referenzkarte, die einen Referenzwert oder einen Bereich von Werten von Aktivitätsniveaus oder -arten innerhalb der bestimmten Zeitschlitze angibt;

Vergleichen (14) des Niveaus oder der Art einer bestimmten Aktivität in den einzelnen Zeitschlitzen der Aktivitätsdichtekarte mit der Referenzverteilung von Aktivitätsniveaus oder -arten in den entsprechenden Zeitschlitzen der Referenzkarte;

Bestimmen einer Größe einer Entsprechung des Niveaus oder der Art von Aktivität, die in jedem Zeitschlitz der Aktivitätsdichtekarte entsteht, mit der Referenzverteilung von Aktivitätsniveaus oder -arten in den entsprechenden Zeitschlitzen der Referenzkarte, um anfängliche Anomalitätspunkte zu identifizieren; **dadurch gekennzeichnet, dass** die Datenverarbeitungseinheit weiter konfiguriert ist zum:

für die anfänglichen Anomalitätspunkte, Durchführen eines Tests auf Aktivitätspermutationen, um Zeitschlitze der Aktivitätsdichtekarte zu finden, die neu geordnet werden können, um so viele der anfänglichen Anomalitätspunkte wie möglich zu entfernen; und
Identifizieren der verbleibenden Anomalitätspunkte als eine erste Anomalitätsangabe.

2. System nach Anspruch 1, wobei die Datenverarbeitungseinheit (134) ausgebildet ist, den Test auf Aktivitätspermutationen durchzuführen durch:

Einstellen eines Zeitfensters, das auf einer anfänglichen Anomalität zentriert ist;
Testen auf Neuordnung von Zeitschlitzen innerhalb des Zeitfensters, die die anfängliche Anomalität entfernen;
Bestimmen, ob die Zeitschlitzneuordnung neue Anomalitäten kreiert oder nicht.

3. System nach Anspruch 2, wobei die Datenverarbeitungseinheit (134) ausgebildet ist, den Test auf Aktivitätspermutationen durch rekursives Testen von Zeitschlitzwechseln innerhalb des Zeitfensters durchzuführen, um die minimale verbleibende Anzahl von Anomalitätspunkten für das Zeitfenster zu finden.

4. System nach einem der vorstehenden Ansprüche, wobei die Aktivitätsdichtekarte und die Referenzkarte einer Zeitspanne eines Satzes vollständiger Tage entsprechen.

5. System nach einem der vorstehenden Ansprüche, wobei die Datenverarbeitungseinheit (134) ausgebildet ist, die Größe einer Entsprechung durch Bestimmen eines Wahrscheinlichkeitswerts des Aktivitätsniveaus, das in jedem Zeitschlitz der Aktivitätsdichtekarte entsteht, basierend auf der Referenzkarte zu bestimmen, und ausgebildet ist, die gesamte Wahrscheinlichkeit zu optimieren.

6. System nach einem der vorstehenden Ansprüche, wobei die Datenverarbeitungseinheit (134) ausgebildet ist, die Referenzkarte als eine Abfolge von Aktivitätswahrscheinlichkeitsverteilungen für jeden Zeitschlitz zu erzeugen.

7. System nach Anspruch 6, wobei die Datenverarbeitungseinheit (134) ausgebildet ist zum:

Bilden eines Rekurrenzplots aus der Abfolge von Aktivitätswahrscheinlichkeitsverteilungen; und
Identifizieren der anfänglichen Anomalitätspunkte als fehlende Punkte aus der Hauptdiagonale des Rekurrenzplots.

8. System nach einem der vorstehenden Ansprüche, wobei die Datenverarbeitungseinheit (134) ausgebildet ist zum:

Identifizieren von Zeitschlitzen, die durchgehend in der Aktivitätsdichtekarte anfänglichen Anomalitätspunkten entsprechen, und Bereitstellen einer zweiten Anomalitätsangabe basierend auf den identifizierten Zeitschlitzen; und
Erhalten einer durchschnittlichen Aktivitätsdichte für die Aktivitätsdichtekarte und Vergleichen der durchschnittlichen Aktivitätsdichte mit der durchschnittlichen Aktivitätsdichte für die Referenzkarte und Bereitstellen einer dritten Anomalitätsangabe basierend auf dem Vergleich.

9. System nach einem der vorstehenden Ansprüche, wobei der Satz von Sensoren (130) eines oder mehrere umfasst von : PIR-Sensoren; Offen/Geschlossen-Sensoren; Leistungssensoren; Mattendrucksensoren; Sensoren auf Radar- und Ultraschallbasis; Feuchtigkeitssensoren; $CO_2$-Sensoren; Temperatursensoren; Mikrofone; Kameras; tragbare Sensoren; Beschleunigungsmesser; Gyroskope; Herzschlagmonitore; Atmungssensoren; Körpertemperatursensoren; Hautleitfähigkeitssensoren; Blutdrucksensoren; Zuckerspiegelsensoren.

10. Computerimplementiertes Verfahren zur Überwachung von Aktivitäten des täglichen Lebens, ADLs, einer Person innerhalb einer Umgebung, umfassend:

mit Hilfe einer Datenverarbeitungseinheit (134),
Empfangen von Sensorausgangssignalen von einem Satz von Sensoren (130), die jeweils ausgebildet sind, auf eine Aktivität anzusprechen und ein Sensorausgangssignal zu erzeugen, das für die Aktivität repräsentativ ist;

Verarbeiten der Sensorausgangssignale zum:

Erzeugen (10) einer Aktivitätsdichtekarte, die das Niveau oder die Art einer bestimmten Aktivität innerhalb bestimmter Zeitschlitze identifiziert;

Erzeugen (12) einer Referenzkarte, die einen Referenzwert oder einen Bereich von Werten von Aktivitätsniveaus oder -arten innerhalb der bestimmten Zeitschlitze angibt;

Vergleichen (14) des Niveaus oder der Art einer bestimmten Aktivität in den einzelnen Zeitschlitzen der Aktivitätsdichtekarte mit der Referenzverteilung von Aktivitätsniveaus oder -arten in den entsprechenden Zeitschlitzen der Referenzkarte;

Bestimmen einer Größe einer Entsprechung des Niveaus oder der Art von Aktivität, die in jedem Zeitschlitz der Aktivitätsdichtekarte entsteht, mit der Referenzverteilung von Aktivitätsniveaus oder -arten in den entsprechenden Zeitschlitzen der Referenzkarte, um anfängliche Anomalitätspunkte zu identifizieren;

**dadurch gekennzeichnet, dass** die Verarbeitung weiter umfasst:

für die anfänglichen Anomalitätspunkte, Durchführen eines Tests auf Aktivitätspermutationen, um Zeitschlitze der Aktivitätsdichtekarte zu finden, die neu geordnet werden können, um so viele der anfänglichen Anomalitätspunkte wie möglich zu entfernen; und

Identifizieren der verbleibenden Anomalitätspunkte als eine erste Anomalitätsangabe.

11. Verfahren nach Anspruch 10, umfassend ein Durchführen des Tests auf Aktivitätspermutationen durch:

Einstellen eines Zeitfensters, das auf einer anfänglichen Anomalität zentriert ist;

Testen auf Neuordnung von Zeitschlitzen innerhalb des Zeitfensters, die die anfängliche Anomalität entfernen;

Bestimmen, ob die Zeitschlitzneuordnung neue Anomalitäten kreiert oder nicht.

12. Verfahren nach Anspruch 11, umfassend ein Durchführen des Tests auf Aktivitätspermutationen durch rekursives Testen von Zeitschlitzwechseln innerhalb des Zeitfensters, um die minimale verbleibende Anzahl von Anomalitätspunkten für das Zeitfenster zu finden.

13. Verfahren nach einem der Ansprüche 10 bis 12, umfassend ein Bestimmen der Größe einer Entsprechung durch Bestimmen eines Wahrscheinlichkeitswerts des Aktivitätsniveaus, das in jedem Zeitschlitz der Aktivitätsdichtekarte entsteht, basierend auf der Referenzkarte, und Optimieren der gesamten Wahrscheinlichkeit.

14. Verfahren nach einem der Ansprüche 10 bis 13, umfassend:

Erzeugen der Referenzkarte als eine Abfolge von Aktivitätswahrscheinlichkeitsverteilungen für jeden Zeitschlitz;

Bilden eines Rekurrenzplots aus der Abfolge von Aktivitätswahrscheinlichkeitsverteilungen; und

Identifizieren der anfänglichen Anomalitätspunkte als fehlende Punkte aus der Hauptdiagonale des Rekurrenzplots.

15. Computerprogramm, umfassend einen Softwarecode, der ausgebildet ist, das System nach Anspruch 1 zu veranlassen, das Verfahren nach einem der Ansprüche 10 bis 14 auszuführen, wenn es auf der Datenverarbeitungseinheit des Systems durchgeführt wird.

**Revendications**

1. Système de surveillance d'une activité de vie quotidienne ADL pour surveiller les ADL d'une personne dans un environnement, dans lequel le système de surveillance d'ADL comprend :

un ensemble de capteurs (130) adaptés chacun pour répondre à une activité et générer un signal de sortie de capteur représentatif de l'activité ;

une unité de traitement de données (134) qui est à même de recevoir les signaux de sortie des capteurs et de traiter les signaux de sortie des capteurs pour :

générer (10) une carte de densité d'activité qui identifie le niveau ou le type d'une activité particulière dans des tranches de temps particulières ;

générer (12) une carte de référence qui indique une valeur de référence ou une plage de valeurs de niveaux ou de types d'activité dans les tranches de temps particulières ;

comparer (14) le niveau ou le type d'une activité particulière dans les tranches de temps individuelles de la carte de densité d'activité avec la fourchette de référence de niveaux ou de types d'activité dans les tranches de temps correspondantes de la carte de référence ;

déterminer une taille de correspondance du niveau ou du type d'activité se produisant dans chaque tranche de temps de la carte de densité d'activité avec la fourchette de référence de niveaux ou de types d'activité dans les tranches de temps correspondantes de la carte de référence afin d'identifier des points d'anomalies initiaux ;

**caractérisé en ce que** l'unité de traitement de données est en outre configurée pour :

pour les points d'anomalie initiaux, effectuer un test de permutations d'activités pour trouver les tranches de temps de la carte de densité d'activité qui peuvent être réaménagées afin d'éliminer autant de points d'anomalie initiaux que possible ; et

identifier les points d'anomalie restants en tant que première indication d'anomalie.

2. Système selon la revendication 1, dans lequel l'unité de traitement de données (134) est à même d'effectuer le test de permutations d'activités :

en réglant une fenêtre temporelle centrée sur une anomalie initiale ;

en testant le réaménagement de tranches de temps de la fenêtre temporelle qui éliminent l'anomalie initiale ;

en déterminant si le réaménagement des tranches de temps crée ou non de nouvelles anomalies.

3. Système selon la revendication 2, dans lequel l'unité de traitement de données (134) est à même d'effectuer le test de permutations d'activités en testant de manière récursive les échanges de tranches de temps de la fenêtre temporelle afin de trouver le nombre minimal restant de points d'anomalie pour la fenêtre temporelle.

4. Système selon l'une quelconque des revendications précédentes, dans lequel la carte de densité d'activité et la carte de référence correspondent à une période de temps d'un ensemble de jours complets.

5. Système selon l'une quelconque des revendications précédentes, dans lequel l'unité de traitement de données (134) est à même de déterminer la taille de correspondance en déterminant une valeur de probabilité du niveau d'activité se produisant dans chaque tranche de temps de la carte de densité d'activité sur la base de la carte de référence et est à même d'optimiser la probabilité totale.

6. Système selon l'une quelconque des revendications précédentes, dans lequel l'unité de traitement de données (134) est à même de générer la carte de référence comme une séquence de distribution de probabilité d'activité pour chaque tranche de temps.

7. Système selon la revendication 6, dans lequel l'unité de traitement de données (134) est à même :

de former un graphique de récurrence à partir de la séquence de distributions de probabilités d'activité ; et

d'identifier les points d'anomalie initiaux comme points manquants dans la diagonale principale du graphique de référence.

8. Système selon l'une quelconque des revendications précédentes, dans lequel l'unité de traitement de données (134) est à même :

d'identifier des tranches de temps qui dans toute la carte de densité d'activité correspondent à des points d'anomalie initiaux et de fournir une seconde indication d'anomalie sur la base des tranches de temps identifiées ; et

d'obtenir une densité d'activité moyenne pour la carte de densité d'activité et de comparer la densité d'activité moyenne à la densité d'activité moyenne pour la carte de référence et de fournir une troisième indication d'anomalie sur la base de la comparaison.

9. Système selon l'une quelconque des revendications précédentes, dans lequel l'ensemble de capteurs (130) comprend un ou plusieurs des suivants : capteurs PIR ; capteurs de type ouvert/fermé ; capteurs de puissance ; capteurs de pression de matériels ; capteurs à base de radar et d'ultrasons ; capteurs d'humidité ; capteurs de $CO_2$ ; capteurs

de température ; microphones ; caméras ; capteurs portables ; accéléromètres ; gyroscopes ; moniteurs de battements cardiaques ; capteurs de respiration ; capteurs de température corporelle ; capteurs de conductivité de la peau ; capteurs de pression sanguine ; détecteurs de niveau de sucre.

10. Procédé mis en oeuvre par ordinateur de surveillance d'activités de vie quotidienne ADL d'une personne dans un environnement, comprenant :

   au moyen d'une unité de traitement de données (134),
   la réception de signaux de sortie d'un ensemble de capteurs (130) adaptés chacun pour répondre à une activité et générer un signal de sortie de capteur représentatif de l'activité détectée ; et
   le traitement des signaux de sortie de capteurs pour :

   générer (10) une carte de densité d'activité qui identifie le niveau ou le type d'une activité particulière dans des tranches de temps particulières ;
   générer (12) une carte de référence qui indique une valeur de référence ou une plage de valeurs de niveaux ou de types d'activité dans les tranches de temps particulières ;
   comparer (14) le niveau ou le type d'une activité particulière dans les tranches de temps individuelles de la carte de densité d'activité avec la fourchette de référence de niveaux ou de types d'activité dans les tranches de temps correspondantes de la carte de référence ;
   déterminer une taille de correspondance du niveau ou du type d'activité se produisant dans chaque tranche de temps de la carte de densité d'activité avec la fourchette de référence de niveaux ou de types d'activité dans les tranches de temps correspondantes de la carte de référence afin d'identifier des points d'anomalies initiaux ;
   **caractérisé en ce que** le traitement comprend en outre les étapes consistant :

   pour les points d'anomalie initiaux, à effectuer un test de permutations d'activités pour trouver les tranches de temps de la carte de densité d'activité qui peuvent être réaménagées afin d'éliminer autant de points d'anomalie initiaux que possible ; et
   à identifier les points d'anomalie restants en tant que première indication d'anomalie.

11. Procédé selon la revendication 10, comprenant la réalisation du test de permutations d'activités :

   en réglant une fenêtre temporelle centrée sur une anomalie initiale ;
   en testant le réaménagement de tranches de temps de la fenêtre temporelle qui éliminent l'anomalie initiale ;
   en déterminant si le réaménagement des tranches de temps crée ou non de nouvelles anomalies.

12. Procédé selon la revendication 11, dans lequel la réalisation du test de permutations d'activités en testant de manière récursive les échanges de tranches de temps de la fenêtre temporelle afin de trouver le nombre minimal restant de points d'anomalie pour la fenêtre temporelle.

13. Procédé selon l'une quelconque des revendications 10 à 12, comprenant la détermination de la taille de correspondance en déterminant une valeur de probabilité du niveau d'activité se produisant dans chaque tranche de temps de la carte de densité d'activité sur la base de la carte de référence et en optimisant la probabilité totale.

14. Procédé selon l'une quelconque des revendications 10 à 13, comprenant :

   la génération de la carte de référence comme une séquence de distributions de probabilité d'activité pour chaque tranche de temps ; et
   la formation d'un graphique de récurrence à partir de la séquence de distributions de probabilité d'activité ; et
   l'identification des points d'anomalie initiaux comme points manquants de la diagonale principale du graphique de récurrence.

15. Programme informatique comprenant un code logiciel qui est à même d'amener le système selon la revendication 1 à effectuer le procédé selon l'une quelconque des revendications 10 à 14 lorsqu'il est exécuté sur l'unité de traitement de données dudit système.

FIG. 1

FIG. 2

FIG. 3

FIG. 4

FIG. 5

FIG. 6(a)

FIG. 6(b)

FIG. 6(c)

FIG. 7(a)

FIG. 7(b)

FIG. 8

FIG. 9

FIG. 10

FIG. 11

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090066501 A1

### Non-patent literature cited in the description

- **J.P.ECKMAN ; S.OLIFFSON KAMPHORSTA.** Recurrence Plots of Dynamical Systems. *Europhys. Lett.,* 1987, vol. 4 (91), 973-977

- **CHARLES L. WEBBER, JR. ; JOSEPH P. ZBILUT.** Dynamical assessment of physiological systems and states using recurrence plot. *Journal of Applied Physiology,* March 1994, vol. 76 (2), 965-73